# DEMANDE DE BREVET EUROPEEN

(11) **EP 3 495 816 A1**
(43) Date de publication de la demande: **12.06.2019**
(21) Numéro de dépôt: 18214693.6
(22) Date de dépôt: 06.04.2012
(51) Int. Cl.: G01N 33/50

(54) **METHODES DE DETECTION DE CONTAMINANTS DANS DES SOLUTIONS CONTENANT DES POLYMERES DE GLUCOSE**

(30) Priorité: 08.04.2011 FR 1153050; 19.05.2011 FR 1154342; 02.08.2011 FR 1157073; 29.11.2011 FR 1160921
(62) Demande divisionnaire de: 12720540.9
(71) Demandeur: Roquette Frères, 62136 Lestrem (FR)
(72) Inventeur: LANOS, Pierre, 59480 La Bassee (FR); HACINE-GHERBI, Héla, 59650 Villeneuve d'Ascq (FR); ALLAIN, Fabrice, 59800 Lille (FR); CARPENTIER, Mathieu, 59350 Saint Andre Lez Lille (FR); DENYS, Agnès, 59800 Lille (FR)
(74) Mandataire: Cabinet Plasseraud

(57) **Abrégé**

L'invention a pour objet un procédé de détection des contaminants des polymères de glucose, contaminants susceptibles d'agir en synergie les uns avec les autres pour déclencher une réaction inflammatoire, caractérisé en ce qu'il comprend un test de réponse inflammatoire *in vitro* à l'aide de lignées cellulaires modifiées.

## Description

La présente invention est relative à des méthodes de détection des contaminants de polymères de glucose, en particulier des circuits de production de polymères de glucose, plus particulièrement ceux destinés à la dialyse péritonéale.

Par extension, ce procédé permet également la détection des contaminants de polymères de glucose, en particulier des circuits de production de polymères de glucose, destinés à la nutrition entérale et parentérale, voire à la nutrition des nouveaux nés.

L'invention a également pour objet les moyens d'identification des contaminants pro-inflammatoires.

### Arrière-plan technologique de l'invention

La société Demanderesse a choisi de développer son invention dans un domaine connu pour la dangerosité des contaminants susceptibles d'être amenés par les polymères de glucose, contaminants à l'origine de réactions inflammatoires très néfastes pour la santé humaine : celui de la dialyse péritonéale.

La dialyse péritonéale est un type de dialyse qui a pour objectif d'éliminer les déchets tels que l'urée, la créatinine, l'excès de potassium ou l'excédent d'eau que les reins ne parviennent pas ou plus à épurer du plasma sanguin. Ce traitement médical est indiqué en cas d'insuffisance rénale chronique terminale.

C'est une épuration intracorporelle qui utilise le péritoine comme membrane de dialyse. Les déchets toxiques du sang traversent la membrane semi-perméable du péritoine, vers une solution appelée dialysat. Le dialysat est introduit dans la cavité péritonéale par un cathéter permanent. Il existe deux types de dialyse péritonéale :
- La DPCA (dialyse péritonéale continue ambulatoire), traitement qui se base sur le passage de 4 poches de dialysat par jour selon prescription médicale
- La DPA (dialyse péritonéale automatisée), traitement nocturne continu qui correspond à environ 15 litres de dialysat par 8 heures selon prescription médicale.

Les dialysats les plus couramment utilisés sont composés d'une solution tampon (du lactate ou du bicarbonate) à pH acide (5,2 - 5,5) ou physiologique (7,4) à laquelle sont ajoutés des électrolytes (sodium, calcium, magnésium, chlore) et un agent osmotique (du glucose ou un polymère de glucose, tel que l'« icodextrine » présent dans la solution pour dialyse péritonéale ambulatoire EXTRANEAL® commercialisée par la société BAXTER).

Le polymère de glucose, tel l'icodextrine mentionné ci-avant, est préféré au glucose comme agent osmotique, car en raison de sa petite taille, le glucose qui traverse rapidement le péritoine mène à la perte de gradient osmotique dans les 2 à 4 heures d'infusion.

Les polymères de glucose standards sont produits par hydrolyse acide ou enzymatique d'amidon de céréales ou de tubercules.

L'hydrolyse acide de l'amidon, totalement aléatoire, ou son hydrolyse enzymatique un peu plus ordonnée, fournissent des mélanges de glucose (monomère) et des chaînes de glucose qui comportent des molécules très courtes (oligomères), de faible Degré de Polymérisation (ou D.P.), aussi bien que des molécules très longues (polymères), de D.P. élevé. Les polymères de glucose ont par ailleurs un poids moléculaire extrêmement varié.

Dans le domaine plus particulier de l'utilisation des polymères du glucose destinés à la dialyse péritonéale continue et ambulatoire, il est très vite apparu que ces hydrolysats d'amidon (mélange de glucose, d'oligomères et de polymères de glucose) ne pouvaient pas être utilisés tels quels.

La demande de brevet européen EP 207.676 enseigne qu'il est préféré des polymères de glucose formant des solutions limpides et incolores à 10 % dans l'eau, ayant un poids moléculaire moyen en poids (Mw) de 5.000 à 100.000 daltons et un poids moléculaire moyen en nombre (Mn) inférieur à 8.000 daltons.

De tels polymères de glucose comprennent aussi de façon préférée au moins 80 % de polymères du glucose dont le poids moléculaire est compris entre 5.000 et 50.000 daltons, peu ou pas de glucose ou de polymères du glucose de DP inférieur ou égal à 3 (poids moléculaire 504) et peu ou pas de polymères de glucose de poids moléculaire supérieur à 100.000 (DP voisin de 600).

En d'autres termes, les polymères de glucose préférés sont des polymères de glucose de faible indice de polymolécularité (valeur obtenue en calculant le rapport Mw/Mn).

Les procédés proposés dans cette demande de brevet EP 207.676 pour obtenir ces polymères de glucose de faible indice de polymolécularité à partir d'hydrolysats d'amidon consistent :
- soit à effectuer une précipitation fractionnée d'une maltodextrine à l'aide d'un solvant miscible à l'eau,
- soit à effectuer une filtration moléculaire de cette même maltodextrine au travers de différentes membranes possédant un seuil de coupure ou d'exclusion adéquat.

Dans les deux cas, ces procédés visent à éliminer à la fois les polymères de très haut poids moléculaire et les monomères ou oligomères de faible poids moléculaire.

Ces procédés ne donnent toutefois pas satisfaction tant du point de vue de leur mise en oeuvre que du point de vue des rendements et de la qualité des produits qu'ils permettent d'obtenir.

Soucieuse de mettre au point un procédé de fabrication d'un polymère de glucose complètement soluble dans l'eau et de faible indice de polymolécularité préférentiellement inférieur à 2,5, ayant de préférence un Mn inférieur à 8.000 daltons et possédant un Mw compris entre 12.000 et 20.000 daltons, procédé qui soit dépourvu des inconvénients de l'art antérieur, la société Demanderesse, s'est attachée à résoudre ce problème dans son brevet EP 667.356, en partant d'un amidon hydrolysé, plutôt que d'une maltodextrine.

Le polymère de glucose obtenu par fractionnement chromatographique contient alors de préférence moins de 3 % de glucose et de polymères de glucose de DP inférieur ou égal à 3 et moins de 0,5 % de polymères de glucose de DP supérieur à 600.

Il est finalement désormais admis par les experts du domaine de la dialyse péritonéale que ces polymères de glucose, utilisés pour leur pouvoir osmotique, donnent toute satisfaction.

Il est cependant à déplorer des risques de contamination microbienne de ces préparations destinées à la dialyse péritonéale.

Il est en effet connu que les circuits de production des polymères de glucose peuvent être contaminés par des microorganismes, ou par des substances pro-inflammatoires contenus dans lesdits microorganismes.

Il est par exemple décrit en amidonnerie la contamination des amidons de maïs ou de blé par des microorganismes de type levures, moisissures et bactéries, et plus particulièrement par des bactéries acidothermophiles de type *Alicyclobacillus acidocaldarius* (bactéries extrémophiles qui se développent dans les zones chaudes et acides du circuit).

Le risque majeur pour le patient qui reçoit ces produits contaminés est alors la péritonite.

Le soupçon clinique de la péritonite est diagnostiqué lors du développement d'un trouble dans le dialysat associé avec les manifestations cliniques variables que sont la douleur abdominale, la nausée, le vomissement, la diarrhée et la fièvre.

Ces épisodes de péritonite sont provoqués par des infections bactériennes intrapéritonéales, et le diagnostic est habituellement facilement établi par les cultures positives de dialysat.

La « péritonite stérile », également décrite en tant que péritonite aseptique, chimique, ou culture-négative, est quant à elle typiquement provoquée par un irritant chimique ou un corps étranger.

Depuis l'introduction de l'icodextrine pour la préparation de solutions de dialyse péritonéale, des cas isolés de péritonite aseptique ont été rapportés, pouvant être liés à des causes diverses et notamment l'induction par des substances pro-inflammatoires potentiellement présentes.

Les épisodes inflammatoires aseptiques sont donc des complications majeures observées après injections de solutions de dialyse.

Si une partie de ces épisodes inflammatoires est liée à un problème d'ordre chimique (injection accidentelle de contaminants chimiques ou mauvais dosages de certains composés), la majorité des cas est directement associée à la présence de contaminants d'origine microbienne présents dans les solutions servant à la préparation des solutions de dialyse.

Les lipopolysaccharides (LPS) et les peptidoglycanes (PGN) sont les principaux contaminants d'origine microbienne présentant un risque élevé de déclencher une inflammation lorsqu'ils sont présents à l'état de trace.

Les tests standards permettent théoriquement d'écarter les lots chargés en contaminants de ce type et présentant donc un risque sanitaire. Toutefois, ces tests ne sont pas satisfaisants, puisque des épisodes inflammatoires aseptiques sont encore reportés, alors que les solutions avaient été déclarées saines.

Ainsi, malgré l'attention constante des acteurs du domaine pour diminuer le risque de contaminations, notamment en améliorant sa détection, il reste toujours un besoin d'améliorer les performances de la détection de contaminants pouvant induire une inflammation.

### Description détaillée de l'invention

Il est du mérite de la société Demanderesse d'avoir pris en compte la présence de molécules susceptibles d'exacerber la réponse inflammatoire induite par d'autres contaminants, en particulier le LPS ou les PGN, notamment par un mécanisme de coopération entre les TLRs (acronyme anglo-saxon de *Toll Like Receptors*) et les récepteurs NODs (*Nucleotide-binding Oligomerization Domain-containing proteins*).. En effet, la seule considération de l'effet des contaminants isolés sur l'inflammation est réductrice.

Contrairement au LPS qui est un ligand reconnu par les récepteurs de type TLR4 (acronyme anglo-saxon de *Toll Like Receptor*), le PGN (mais également de nombreux glycolipides et lipopeptides) est un ligand reconnu par les récepteurs de type TLR2 qui induit une réponse inflammatoire faible dans les modèles *in vitro* et *in vivo,* ce qui implique que ces molécules doivent être présentes à des concentrations plus importantes pour être détectées.

Ainsi, dans des modèles utilisant des cellules mononucléaires (PBMC, monocytes/macrophages primaires ou lignées monocytaires), le LPS induit une réponse significative pour des concentrations de l'ordre du ng/mL, alors que des concentrations au moins 100 fois plus élevées en PGN sont nécessaires pour obtenir une réponse similaire (ratio P/P).

En outre, alors que les PGN solubles (MM ≈ 125 kDa) induisent une réponse inflammatoire *via* l'activation de TLR2, ses produits de dépolymérisation, dont la structure minimale encore bioactive est le muramyl-dipeptide (MDP), interagissent avec des récepteurs intracellulaires de type NOD.

Ces dérivés, considérés isolément, sont peu inflammatoires *in vitro* et donnent une réponse significative pour des valeurs > 1 µg/mL.

Par contre, la présence de ces molécules a un effet synergique sur la réponse inflammatoire, par un mécanisme de coopération entre les TLRs et les récepteurs NOD, et ce, quelque soit le modèle expérimental utilisé (souris, lignées de monocytes/macrophages, cellules mononucléaires du sang).

En plus des produits de dépolymérisation du PGN, les peptides microbiens formylés, dont le prototype est le f-MLP (tripeptide formyl-Met-Leu-Phe), ont également une activité synergique importante. A l'origine, ces peptides ont été identifiés pour leur activité chimio-attractante sur les leucocytes, alors qu'ils sont incapables d'induire une réponse cytokinique *per se.*

Cependant, lorsqu'ils sont associés à des agonistes des TLRs, ils contribuent à augmenter la production de cytokines en sensibilisant les cellules cibles.

Il est donc important de ne pas négliger ces "petites molécules", car elles peuvent rendre compte indirectement des épisodes inflammatoires aseptiques en exacerbant les effets de traces de PGN et/ou de LPS.

Ces dernières années, de nombreux tests utilisant des cellules primaires se sont développés pour remplacer les modèles animaux dans les tests de réponse inflammatoire.

Toutefois, ces modèles *in vitro* sont sujets à une importante variabilité interindividuelle, ce qui peut être responsable de biais expérimentaux.

A l'inverse, les lignées cellulaires monocytaires donnent des réponses constantes, ce qui explique pourquoi les tests actuellement en développement utilisent de plus en plus ce type de cellules en culture. Cependant, ces tests présentent l'inconvénient de donner une réponse inflammatoire globale à tous les contaminants présents en mélange dans une solution, et par conséquent ne permettent pas de caractériser la nature du contaminant.

Il est également important de noter que la réponse inflammatoire exacerbée est visible pour les cytokines de la phase aiguë de l'inflammation, telles que TNF-α, (Tumor Necrosis Factor alpha), l'IL-1β (interleukine 1β) et les chimiokines telles que CCL5 (Chemokine (C-C motif) ligand 5) /RANTES (Regulated upon Activation, Normal T-cell Expressed, and Secreted), mais pas ou peu pour l'IL-6 (interleukine 6). Ainsi, les méthodes basées sur la production de cette dernière (US2009/0239819 et US2007/0184496) ne sont adaptées pour détecter des contaminants en mélange dans une solution.

Ainsi, la société Demanderesse a abouti aux conclusions suivantes:
*(i)* il est difficile de détecter des contaminants bactériens présents à l'état de traces dans des solutions biologiques,
*(ii)* il est important de ne pas se limiter à la détection des PGN et du LPS, en raison des effets synergiques,
*(iii)* il est nécessaire de développer de nouvelles méthodes de détection sensibles et reproductibles, et
*(iv)* il est avantageux d'utiliser des méthodes de détection sensibles et reproductibles, capables de caractériser la nature des contaminants.

Il est donc du mérite de la société Demanderesse d'avoir développé des méthodes sensibles et efficaces de détection des contaminants microbiens ayant une action pro-inflammatoire, en deçà du seuil de sensibilité des procédures actuellement utilisées et/ou décrites dans la littérature, et ultérieurement d'identifier la famille, voire la nature, des molécules pro-inflammatoires présentes sous formes de traces dans les lots provenant des circuits de production.

En effet, une méthode très sensible de mesure des réponses inflammatoires *in vitro* permettra de retenir ou non des lots sur la base de taux de contamination "non significatifs", au sens où ces taux seront inférieurs aux niveaux actuellement mesurables par les tests standards.

Ces lots pourront être proposés pour entrer dans la composition de solutions à usage thérapeutique chez l'Homme (*e.g*. solutions de dialyse péritonéale).

De plus, l'identification des molécules responsables des réponses inflammatoires devra permettre de détecter les sources de contaminations au cours des procédés de fabrication, et d'apporter des modifications correctrices pour diminuer les niveaux de contaminants, voire les éliminer.

Le procédé conforme à l'invention porte donc sur un procédé de détection des contaminants pro-inflammatoires des polymères de glucose, en particulier ceux destinés à la préparation de solution pour dialyse péritonéale, comprenant un test de réponse inflammatoire *in vitro.*

Les polymères de glucose peuvent être destinés à la dialyse péritonéale, la nutrition entérale et parentérale et l'alimentation des nouveaux nés. Dans un mode de réalisation préféré, les polymères de glucose qui seront testés par les méthodes de la présente invention sont de l'icodextrine ou des maltodextrines. Ils peuvent être testés à un ou plusieurs stades de leur préparation, et notamment au niveau de la matière première, à une étape quelconque de leur procédé de préparation, et/ou au niveau du produit final du procédé. Ils peuvent également être testés en tant qu'échantillon d'une solution de dialyse péritonéale.

Comme mentionné précédemment, certaines molécules d'origine bactérienne, tels que le MDP et le f-MLP, sont de faibles inducteurs inflammatoires, mais ils peuvent agir en combinaison ou en synergie et augmenter la réponse induite par d'autres contaminants.

Cette propriété repose sur le fait que ces molécules agissent *via* l'entremise de récepteurs autres que les TLRs.

Hormis le LPS qui réagit avec TLR4, la majorité des molécules à potentiel inflammatoire susceptibles d'être présents dans les lots sont des agonistes de TLR2.

Ces contaminants sont difficilement détectables, car ils sont présents en faibles concentrations et la réponse inflammatoire qu'ils vont déclencher est le plus souvent proche du bruit de fond.

Par conséquent, la présence de molécules à activité synergique peut exacerber la réponse inflammatoire induite par les ligands des TLR2, ce qui peut être mis à profit pour détecter de faibles doses de contaminants.

Des échantillons contaminés en MDP (agoniste de NOD2), f-MLP (ligand d'un récepteur à peptide microbien), voire en LPS (pour déclencher une synergie TLR4/TLR2) vont de ce fait déclencher une réponse inflammatoire *in vitro.*

Ainsi, les contaminants pro-inflammatoires détectés par le procédé de l'invention sont susceptibles de déclencher, séparément ou en combinaison, une réaction inflammatoire. De manière particulière, ces contaminants peuvent être, lorsqu'ils sont considérés séparément, de faibles inducteurs inflammatoires mais induire une réaction inflammatoire importante lorsqu'ils sont en combinaison. Le procédé selon l'invention permet de considérer l'effet de l'ensemble des contaminants présents dans la préparation de polymères de glucose considérée et pas uniquement l'effet particulier de chacun d'eux.

Le procédé selon l'invention comprend au moins un test de réponse inflammatoire *in vitro* à l'aide d'une lignée cellulaire permettant de détecter au moins un facteur de la réponse inflammatoire. De préférence, la lignée cellulaire est soit un macrophage ou une lignée cellulaire différenciée en macrophage, soit une cellule exprimant un ou plusieurs récepteurs TLR ou NOD tels que TLR2, TLR4 ou NOD2, soit une combinaison de celles-ci.

Selon un premier mode de réalisation, la lignée cellulaire utilisée dans le test de réponse inflammatoire est un macrophage ou une lignée cellulaire différenciée en macrophage. En particulier, la lignée cellulaire est productrice de TNF-α et de la chimiokine CCL5 /RANTES. De préférence, le test est réalisé avec des cellules THP-1 différenciées en macrophages.

Dans un mode de réalisation préféré, les macrophages ou cellules différenciées en macrophage, notamment les cellules THP-1 différenciées en macrophages, sont mis en oeuvre à une densité comprise entre 0,5 à 1 x 10⁶ cellules/mL de milieu de culture, de préférence comprise entre 0,7 à 0,8 x 10⁶ cellules/mL, et de manière encore plus préférée environ 0,75 x 10⁶ cellules/mL.

Le test de réponse inflammatoire *in vitro* peut être basé sur la production de TNF-α et/ou de la chimiokine CCL5/RANTES par des macrophages, et notamment les cellules THP-1 différenciées en macrophages, étant donné que l'effet synergique (effet obtenu par la combinaison des différents contaminants) est surtout marqué pour les cytokines de la phase aiguë de l'inflammation (TNF-α, IL-1β, chimiokines), mais pas pour des cytokines de la phase retardée, telles que l'IL-6.

Ainsi, selon un mode de réalisation particulier, le test de réponse inflammatoire consiste à mettre les cellules de la lignée cellulaire, de préférence des macrophages, en présence d'une préparation de polymères de glucose susceptibles de contenir des contaminants pro-inflammatoires et à mesurer la production de cytokines de la phase aigüe de l'inflammation, notamment TNF-α, IL-1β, et/ou des chimiokines, notamment CCL5/RANTES, la production de ces cytokines indiquant que la préparation contient des contaminants susceptibles de déclencher une réaction inflammatoire. Dans un mode de réalisation tout particulièrement préféré, le test comprend la mesure de la production de TNF-α et/ou de CCL5/RANTES, de préférence de CCL5/RANTES. Les dosages des cytokines peuvent être faits par tout moyen bien connu de l'homme du métier, et notamment par ELISA. Dans un mode de réalisation préféré, le test comprend la mesure de la production de TNF-α après 8 h de stimulation. Dans un autre mode de réalisation préféré, le test comprend la mesure de la production de RANTES après 20 h de stimulation, notamment par un dosage ELISA.

Afin d'augmenter la réponse cellulaire induite par des contaminants pro-inflammatoires, par exemple par des LPS et/ou des PGN, un composant permettant d'agir en synergie avec les contaminants peut être ajouté l'échantillon à tester. En effet, cela peut permettre de détecter de plus faible doses de contaminants. De préférence, ce composant peut être le MDP ou une molécule apparentée (N-glycolyl-MDP, L18-MDP), un peptide microbien formylé (f-MLP), ou le LPS. De préférence, ce composant est le MDP, le fMLP ou le LPS. De manière encore plus préférée, ce composant est le MDP ou le LPS. En particulier, le LPS est un LPS de *E. coli.*

Dans un mode de réalisation préféré, le MDP, en particulier le MDP de *S. aureus,* est ajouté à l'échantillon. De préférence, le MDP est ajouté à l'échantillon à une concentration de plus de 1 µg/mL, de préférence à une concentration comprise entre 1 et 100 µg/mL. Dans un mode de réalisation tout particulièrement préféré, le MDP est ajouté à l'échantillon à une concentration de 10 µg/mL.

Dans un autre mode de réalisation préféré, le f-MLP est ajouté à l'échantillon à une concentration de plus de 10 nM, de préférence d'au moins 50, 100, 150, 200, 300, 400 ou 500 nM.

Dans encore un autre mode de réalisation préféré, le LPS, en particulier un LPS de *E. coli,* peut être ajouté à l'échantillon à une concentration d'au moins 10 pg/ml, par exemple à une concentration de 25 pg/mL.

Dans un mode de réalisation préféré, la préparation de polymères de glucose testée présente une concentration de polymères de glucose de 5 à 50 mg/mL, de préférence entre 5 et 10, 20, 30 ou 40 mg/mL. Dans un mode de réalisation particulier, la préparation de polymères de glucose testée présente une concentration de polymères de glucose d'environ 5 mg/mL. Dans un mode de réalisation préféré, la préparation de polymères de glucose testée présente une concentration de polymères de glucose d'environ 25 mg/mL.

Facultativement, un échantillon de la préparation de polymères de glucose peut être traité par une mutanolysine préalablement au test. Cette enzyme, par son activité muramidase, est capable de dépolymériser les PGN. Par exemple, l'enzyme à une concentration d'environ 2500 U/ml peut être mise en présence de l'échantillon, éventuellement dilué pour avoir une concentration en polymère de glucose de 7,5 à 37,5 % (poids/volume), pendant 6 à 16 h, de préférence environ 16 h. Ensuite, l'échantillon ainsi traité sera soumis au test avec des macrophages selon la présente invention. Facultativement, le résultat, c'est-à-dire la production de cytokine, obtenu avec l'échantillon traité par une mutanolysine pourra être comparé au résultat obtenu sans traitement.

Facultativement, dans une autre alternative, l'échantillon de la préparation de polymères de glucose peut être filtré préalablement au test. Le but de cette filtration est essentiellement d'enlever les molécules de haut poids moléculaire, comme les PGN de haut poids moléculaire, et de procéder au test sur le filtrat pour analyser tout particulièrement les contaminants de petites tailles. Le seuil de coupure pour la filtration peut par exemple être compris entre 30 kD et 150 kD, de préférence entre 30 et 100 kD ou entre 30 et 50 kD, et notamment d'environ 30 kD. De préférence, la filtration est faite par une ultrafiltration. Elle peut également être réalisée par tout moyen connu de l'homme du métier. Ainsi, l'échantillon ainsi filtré, le filtrat, sera soumis au test avec des macrophages selon la présente invention. Facultativement, le résultat, c'est-à-dire la production de cytokine, obtenu avec le filtrat pourra être comparé au résultat obtenu sans ou avant filtration. Cela permettra de déduire la contribution inflammatoire spécifique des molécules de petites tailles.

Dans un mode de réalisation préféré et particulier, le procédé présente une ou plusieurs des caractéristiques suivantes :
- la lignée cellulaire est mise en oeuvre à une densité comprise entre 0,5 à 1 x 10⁶ cellules/mL, de préférence comprise entre 0,7 à 0,8 x 10⁶ cellules/mL, et de manière encore plus préférée environ 0,75 x 10⁶ cellules/mL; et/ou
- la concentration de polymère de glucose est inférieure à 50 mg/ml, de préférence environ 25 mg/ml ; et/ou
- on mesure la production de cytokines après 20 h de stimulation pour RANTES et/ou 8 h de stimulation pour TNF-α; et/ou
- on ajoute à la préparation de polymères de glucose du MDP à une concentration comprise entre 1 et 100 µg/mL, de préférence d'environ 10 µg/mL.

De préférence, le procédé comprend toutes les caractéristiques.

Ainsi, dans un mode très particulier, la méthode comprend :
- la mise en présence pendant environ 20 h de macrophages avec une préparation de polymères de glucose susceptibles de contenir des contaminants pro-inflammatoires en présence de MDP, de préférence à une concentration comprise entre 1 et 100 µg/mL, la concentration de polymère de glucose étant inférieure à 50 mg/ml, de préférence environ 25 mg/ml, et les macrophages ayant une densité comprise entre 0,5 à 1 x 10⁶ cellules/mL, de préférence comprise entre 0,7 à 0,8 x 10⁶ cellules/mL, et de manière encore plus préférée environ 0,75 x 10⁶ cellules/mL; et
- la mesure la production de CCL5/RANTES, la production de CCL5/RANTES indiquant que la préparation contient des contaminants susceptibles de déclencher une réaction inflammatoire.

Tout particulièrement, ce premier mode de réalisation permet de détecter la contamination du polymère de glucose par des PGN et/ou LPS, de préférence par des PGN de taille moyenne (notamment d'environ 120 kDa) et/ou LPS, plus particulièrement encore par des LPS.

En particulier, la méthode peut comprendre la quantification des contaminants. Par exemple, cette quantification peut être réalisée à l'aide d'une courbe dose-réponse. Cette courbe dose-réponse peut notamment être réalisée avec les mêmes cellules, dans les mêmes conditions, avec des doses croissantes de contaminants. De préférence, une telle courbe dose-réponse peut être réalisée avec des doses croissantes de LPS.

Ce premier mode de réalisation peut être mise en oeuvre dans les méthodes selon la présente invention seul ou en combinaison avec un second mode de réalisation.

Selon le second mode de réalisation, la lignée cellulaire utilisée pour réaliser le test d'inflammation *in vitro,* est une lignée permettant de détecter l'activité d'un ou plusieurs récepteurs de l'immunité innée.

De manière particulière, cette lignée cellulaire peut être obtenue par transfection stable avec un ou plusieurs vecteurs codant pour un ou plusieurs récepteurs de l'immunité innée.

L'activité d'un récepteur de l'immunité innée peut être détectée, par exemple, en utilisant un gène rapporteur qui est sous la dépendance directe de la voie de signalisation associée audit récepteur. De manière préférée, ce gène rapporteur code pour une protéine colorée ou fluorescente ou pour une protéine dont l'activité peut être mesurée avec ou sans substrat. De manière particulière, le gène rapporteur code pour une phosphatase alcaline.

Ainsi, la méthode comprend la mise en présence d'une ou plusieurs lignées cellulaires exprimant un ou plusieurs récepteurs TLR ou NOD avec la préparation de polymères de glucose et la mesure de l'activité du ou des récepteurs, notamment par l'intermédiaire du signal d'un gène rapporteur. Ce signal du gène rapporteur indique la présence dans la préparation d'un contaminant qui est un agoniste du récepteur.

De manière préférée, la lignée cellulaire permet de détecter l'activité d'un ou plusieurs récepteurs TLR ou NOD, tels que les récepteurs TLR2, 3, 4, 5, 7, 8 ou 9 ou NOD2. De préférence, la lignée cellulaire permet de détecter l'activité d'un ou plusieurs récepteurs choisis parmi TLR2, TLR4 et NOD2. Dans un mode de réalisation particulier, la lignée cellulaire exprime les récepteurs TLR2, TLR4 et NOD2 et permet de détecter leur activité.

Les lignées cellulaires utilisées peuvent être par exemple des lignées HEK-Blue™ (commercialisée par la société InvivoGen), modifiées par transfection stable avec des vecteurs codant pour des récepteurs de l'immunité innée. Cependant, il est à noter que l'homme du métier peut également utiliser d'autres lignées commercialement (Imgenex) ou il peut en préparer.

Ces cellules peuvent être également co-transfectées avec un gène rapporteur produisant, par exemple, une forme sécrétée de la phosphatase alcaline (acronyme anglo-saxon SEAP: *secreted embryonic alkaline phosphatase*), dont la synthèse est sous la dépendance directe de la voie de signalisation associée au(x) récepteur(s) exprimé(s) dans la même lignée cellulaire. Dans un mode de réalisation préféré, la réaction enzymatique est mise en oeuvre en utilisant un ratio 1 : 3 de milieu à tester versus le réactif SEAP (par exemple 50 µL de milieu et 150 µL de réactif SEAP). En outre, un temps de réaction d'au moins 60 minutes sera préféré.

Les lignées cellulaires peuvent, par exemple, être choisies dans le groupe constitué de :
- la lignée HEK-Blue™ hTLR2 (lignée qui répond spécifiquement aux agonistes du TLR2),
- la lignée HEK-Blue™ hNOD2 (lignée qui répond efficacement aux produits de dépolymérisation du PGN et aux molécules apparentées (MDP, L18-MDP, ...) et
- la lignée Raw-Blue™ (lignée de macrophages de souris transfectée pour exprimer une phosphatase alcaline). La lignée Raw-Blue™ exprime les récepteurs de l'immunité innée, et particulièrement les récepteurs TLR2, TLR4 et NOD2.

Ces lignées sont détaillées plus avant dans cette description.

Dans un mode de réalisation préférée, une lignée exprimant TLR2 et permettant de détecter son activité, et/ou une lignée exprimant les récepteurs TLR2, TLR4 et NOD2 et permettant de détecter leur activité seront mises en oeuvre. A titre d'exemple, les lignées cellulaires HEK-Blue™ hTLR2 et/ou lignée Raw-Blue™ seront mises en oeuvre. Tout particulièrement, la méthode mettra en oeuvre un test utilisant les lignées cellulaires HEK-Blue™ hTLR2 et Raw-Blue™.

Dans un autre mode de réalisation préféré, deux lignées exprimant respectivement TLR2 et NOD2 et permettant de détecter leur activité (séparément), et une lignée exprimant les récepteurs TLR2, TLR4 et NOD2 et permettant de détecter leur activité seront mises en oeuvre. A titre d'exemple, les lignées cellulaires HEK-Blue™ hTLR2, HEK-Blue™ hNOD2 et Raw-Blue™ seront mises en oeuvre. Tout particulièrement, la méthode mettra en oeuvre un test utilisant les lignées cellulaires HEK-Blue™ hTLR2, HEK-Blue™ hNOD2 et Raw-Blue™.

L'utilisation de telles lignées permet donc de remplacer les dosages des cytokines par un test enzymatique (activité phosphatase), et de cibler certaines familles de molécules d'origine bactérienne en fonction du(es) récepteur(s) exprimé(s) par la lignée.

En outre, ces lignées permettent de détecter des contaminants à des seuils très bas, notamment pour les agonistes de TLR2 (PGN, LTA (acide lipotéichoïque), LM (Lipomannane) ...) et NOD2 (produits de dépolymérisation du PGN et MDP). Ainsi, la lignée exprimant NOD2, en particulier HEK-Blue™ hNOD2, permet tout particulièrement de détecter une contamination par des produits de dépolymérisation du PGN et le MDP, de préférence le MDP. La lignée exprimant TLR2, en particulier HEK-Blue™ hTLR2 et/ou Raw-Blue™, permet tout particulièrement de détecter une contamination par des PGN.

Selon ce second mode de réalisation, le test de réponse inflammatoire *in vitro* consiste à mettre les cellules de la lignée cellulaire permettant de détecter l'activité d'un ou plusieurs récepteurs de l'immunité innée, en présence d'une préparation de polymères de glucose susceptibles de contenir des contaminants pro-inflammatoires et à mesurer l'activité du récepteur ou du signal du gène rapporteur qui lui est associé.

La détection de cette activité ou de ce signal indique que la préparation contient des contaminants susceptibles d'activer un ou des récepteurs de l'immunité innée et de déclencher une réaction inflammatoire.

Dans un mode de réalisation préféré, la préparation de polymères de glucose testée présente une concentration de polymères de glucose de 5 à 50 mg/mL, de préférence entre 5 et 10, 20, 30 ou 40 mg/mL. Dans un mode de réalisation particulier, la préparation de polymères de glucose testée présente une concentration de polymères de glucose d'environ 5 mg/mL. Dans le mode de réalisation préféré, la préparation de polymères de glucose testée présente une concentration de polymères de glucose d'environ 37,5 mg/mL lorsque des cellules HEK-Blue™ hTLR2 et/ou HEK-Blue™ hNOD2 sont mises en oeuvre. Dans un autre mode de réalisation préféré, la préparation de polymères de glucose testée présente une concentration de polymères de glucose d'environ 50 mg/mL lorsque des cellules Raw-Blue™ sont mises en oeuvre.

Facultativement, un échantillon de la préparation de polymères de glucose peut être traité par une mutanolysine préalablement au test. Cette enzyme, par son activité muramidase, est capable de dépolymériser les PGN. Par exemple, l'enzyme à une concentration d'environ 2500 U/ml peut être mise en présence de l'échantillon, éventuellement dilué pour avoir une concentration en polymère de glucose de 7,5 à 37,5 % (poids/volume), pendant 6 à 16 h, de préférence environ 16 h. Ensuite, l'échantillon ainsi traité sera soumis aux méthodes selon le second mode de réalisation. Facultativement, le résultat obtenu avec l'échantillon traité par une mutanolysine pourra être comparé au résultat obtenu sans traitement.

Facultativement, dans une autre alternative, l'échantillon de la préparation de polymères de glucose peut être filtré préalablement au test. Le but de cette filtration est essentiellement d'enlever les molécules de haut poids moléculaire, comme les PGN de haut poids moléculaire, et de procéder au test sur le filtrat pour analyser tout particulièrement les contaminants de petites tailles. Le seuil de coupure pour la filtration peut par exemple être compris entre 30 kD et 150 kD, de préférence entre 30 et 100 kD ou entre 30 et 50 kD, et notamment d'environ 30 kD. De préférence, la filtration est faite par une ultrafiltration. Elle peut également être réalisée par tout moyen connu de l'homme du métier. Ainsi, l'échantillon ainsi filtré, le filtrat, sera soumis aux méthodes selon le second mode de réalisation. Facultativement, le résultat obtenu avec le filtrat pourra être comparé au résultat obtenu sans ou avant filtration. Cela permettra de déduire la contribution inflammatoire spécifique des molécules de petites tailles.

Dans un mode de réalisation préféré et particulier de ce second mode de réalisation, le procédé présente une ou plusieurs des caractéristiques suivantes :
- la lignée cellulaire est mise en oeuvre à une densité d'environ 50000 cellules/puits pour une plaque de 96 puits et pour HEK-Blue™ hTLR2 ou Raw-Blue™ et de 10000 cellules/puits pour une plaque de 96 puits pour HEK-Blue™ hNOD2; et/ou
- la préparation de polymères de glucose testée présente une concentration de polymères de glucose de 5 à 50 mg/mL, de préférence une concentration de polymères de glucose d'environ 37,5 mg/mL lorsque des cellules HEK-Blue™ hTLR2 et/ou HEK-Blue™ hNOD2 sont mises en oeuvre et une concentration de polymères de glucose d'environ 50 mg/mL lorsque des cellules Raw-Blue™ sont mises en oeuvre ; et/ou
- La mise en contact de la préparation de polymères de glucose avec les cellules dure environ 16 à 24 h ; et/ou
- le signal du gène rapporteur SEAP est détecté avec un ratio surnageant de culture : substrat de la SEAP de 20 : 180, de préférence de 50 : 150, après de préférence au moins 60 minutes d'incubation, idéalement 60 minutes.

Dans un mode de réalisation particulier, le procédé comprend toutes les caractéristiques.

En particulier, la méthode peut comprendre la quantification des contaminants. Par exemple, cette quantification peut être réalisée à l'aide d'une courbe dose-réponse. Cette courbe dose-réponse peut notamment être réalisée avec les mêmes cellules, dans les mêmes conditions, avec des doses croissantes de contaminants. De préférence, une telle courbe dose-réponse peut être réalisée pour les cellules exprimant TLR2 (par exemple, HEK-Blue™ hTLR2 et Raw-Blue™) avec des doses croissantes de PGN et pour les cellules exprimant NOD2 (par exemple, HEK-Blue™ hNOD2) avec des doses croissantes de MDP.

Le procédé selon l'invention peut ainsi comprendre en outre une étape consistant à identifier le(s) contaminant(s) susceptible(s) de déclencher une réaction inflammatoire.

Pour cela, une lignée cellulaire permettant de détecter l'activité d'un récepteur ou plusieurs récepteurs de l'immunité innée telle que décrite ci-dessus, est mise en présence de la préparation de polymères de glucose à tester. L'activité du récepteur ou le signal du gène rapporteur associé à ce récepteur est mesuré. La détection de cette activité ou de ce signal indique la présence dans la préparation d'un contaminant qui est un agoniste du récepteur.

Ainsi, la lignée exprimant NOD2, en particulier HEK-Blue™ hNOD2, permet tout particulièrement de détecter une contamination par des produits de dépolymérisation du PGN et le MDP, de préférence le MDP. La lignée exprimant TLR2, en particulier HEK-Blue™ hTLR2 et/ou Raw-Blue™, permet tout particulièrement de détecter une contamination par des PGN. Par ailleurs, les macrophages, en particulier les macrophages de THP-1, permet tout particulièrement de détecter une contamination par des LPS.

Selon un mode de réalisation, le procédé selon l'invention comprend les étapes consistant à
(a) réaliser au moins un test de réponse inflammatoire *in vitro,* tel que décrit ci-dessus dans le premier mode de réalisation, consistant à mettre les cellules d'une lignée cellulaire, de préférence des macrophages, en présence d'une préparation de polymères de glucose susceptibles de contenir des contaminants pro-inflammatoires et à mesurer la production de cytokines de la phase aigue de l'inflammation, notamment TNF-α, IL-1β, et/ou des chimiokines telles que CCL5/RANTES, la production de ces cytokines indiquant que la préparation contient des contaminants susceptibles de déclencher une réaction inflammatoire, et/ou
(b) mettre en présence une lignée cellulaire permettant de détecter l'activité d'un récepteur ou plusieurs récepteurs de l'immunité innée telle que décrite ci-dessus dans le second mode de réalisation, avec la préparation et détecter l'activité du récepteur ou le signal du gène rapporteur associé à ce récepteur, la détection de cette activité ou de ce signal indiquant la présence dans la préparation d'un contaminant qui est un agoniste du récepteur.

Selon un mode de réalisation préféré, le procédé selon l'invention comprend les étapes consistant à
(a) réaliser au moins un test de réponse inflammatoire *in vitro* consistant à mettre les cellules d'une lignée cellulaire, de préférence des macrophages, en présence d'une préparation de polymères de glucose susceptibles de contenir des contaminants pro-inflammatoires et à mesurer la production de cytokines de la phase aigue de l'inflammation, notamment TNF-α, IL-1β, et/ou des chimiokines telles que CCL5/RANTES, la production de ces cytokines indiquant que la préparation contient des contaminants susceptibles de déclencher une réaction inflammatoire, et
(b) dans le cas où la préparation contient des contaminants, mettre en présence une lignée cellulaire permettant de détecter l'activité d'un récepteur ou plusieurs récepteurs de l'immunité innée telle que décrite ci-dessus, avec la préparation et détecter l'activité du récepteur ou le signal du gène rapporteur associé à ce récepteur, la détection de cette activité ou de ce signal indiquant la présence dans la préparation d'un contaminant qui est un agoniste du récepteur.

Les étapes a) et b) de cette méthode sont réalisées selon les détails fournis ci-dessus. De préférence, la méthode comprend les deux étapes.

Dans un mode de réalisation préférée, une lignée cellulaire exprimant le récepteur TLR2 et permettant de détecter son activité, telle que HEK-Blue™ hTLR2, et/ou une lignée exprimant plusieurs récepteurs de l'immunité innée telle que décrite ci-dessus, notamment TLR2, TLR4 et NOD2, telle que Raw-Blue™, seront mises en oeuvre. Tout particulièrement, la méthode mettra en oeuvre un test utilisant les lignées cellulaires HEK-Blue™ hTLR2 et Raw-Blue™.

Dans un autre mode de réalisation préférée, une lignée cellulaire exprimant le récepteur TLR2 et permettant de détecter son activité, telle que HEK-Blue™ hTLR2, une lignée cellulaire exprimant le récepteur NOD2 et permettant de détecter son activité, telle que HEK-Blue™ hNOD2, et/ou une lignée exprimant plusieurs récepteurs de l'immunité innée telle que décrite ci-dessus, notamment TLR2, TLR4 et NOD2, telle que Raw-Blue™, seront mises en oeuvre. Tout particulièrement, la méthode mettra en oeuvre un test utilisant les lignées cellulaires HEK-Blue™ hTLR2, HEK-Blue™ hNOD2 et Raw-Blue™.

Ce procédé permet donc non seulement de détecter la présence de contaminants pro-inflammatoires dans une préparation de polymères de glucose, mais également d'obtenir des informations sur la nature de ces contaminants. Il est notamment possible de définir si ces contaminants sont des agonistes de récepteurs TLR ou NOD tels que TLR2, TLR4 ou NOD2. Selon un mode de réalisation préféré, plusieurs lignées peuvent être utilisées pour apporter des informations complémentaires à celles obtenues, par exemple, avec les tests de réponses cytokiniques dans les cellules THP-1 différenciées :
- lignée HEK-Blue™ hTLR4 : cette lignée répond spécifiquement aux agonistes du TLR4. Elle permet notamment le dosage des LPS.
- lignée HEK-Blue™ hTLR2 : cette lignée répond spécifiquement aux agonistes du TLR2. Elle permet notamment le dosage des PGN.

Son utilisation permet donc de connaître la part de ces contaminants dans le déclenchement des réponses inflammatoires.

Dans un mode de réalisation particulier, l'échantillon de la préparation de polymères de glucose peut être filtré tel que décrit ci-dessus, de manière préférée avec un seuil de coupure à 30 kDa, notamment par ultrafiltration, pour éliminer les PGN, en particulier les PGN de grande taille. Ainsi, les lipopeptides et les glycopeptides de petite taille, qui sont d'autres agonistes de TLR2, sont conservés dans le filtrat et on ne mesurera en testant le filtrat que leur réponse.

En outre, le traitement des solutions par le lysozyme et/ou la β-glucanase permet d'éliminer le PGN et/ou les β-glucanes, et de connaître ainsi l'importance des autres agonistes du TLR2 susceptibles d'être présents dans les lots contaminés (glycolipides et lipopeptides). Par ailleurs, un échantillon de la préparation de polymères de glucose peut être traité par une mutanolysine préalablement au test, notamment comme détaillé ci-dessus.
- lignée HEK-Blue™ hNOD2 : cette lignée répond spécifiquement aux agonistes du NODS. Elle permet notamment le dosage des MDP.

L'utilisation de cette lignée permet donc de les détecter à des concentrations faibles. Cette analyse est d'autant plus intéressante que la présence de PGN suppose que ses produits de dégradation soient aussi présents, et qu'ils puissent agir de façon synergique avec les agonistes des TLRs.
- lignée HEK-Blue™ Null2: il s'agit d'une lignée contrôle, dont l'utilisation est nécessaire pour vérifier que les solutions de polymères de glucose n'induisent pas la production de l'enzyme par un mécanisme intrinsèque.
- lignée Raw-Blue™ : il s'agit d'une lignée de macrophages de souris transfectée par la SEAP.

L'avantage de cette lignée est qu'elle exprime de façon naturelle la quasi-totalité des récepteurs de l'immunité innée.

Elle sert de témoin positif dans les tests, puisqu'elle est censée répondre à tout type de contaminants microbiens.

L'invention a également pour objet les moyens d'identification des contaminants pro-inflammatoires.

Des dosages des LPS et PGN peuvent être réalisés par tout moyen connu de l'homme du métier. Par exemple, la détermination de la teneur en peptidoglycanes peut être réalisée selon deux tests :
- test SLP standard commercialisé par la société WAKO Pure Chemical Industries Ltd.,
- test SLP-HS, test de haute sensibilité mis au point et validé par la société Demanderesse tel que détaillé dans sa demande de brevet WO 2010/125315.

Ces tests possédant des réactifs différents (réactifs SLP, standards PGN) présentent des réactivités biologiques aux impuretés PGN sensiblement différentes, ainsi des critères de performance différents :
- Test SLP standard : SLP reagent n°297-51501-standard PGN *Micrococcus Luteus* n°162-18101
- Test SLP-HS (haute sensibilité) : SLP-HS kit n°293-58301 (incluant un standard PGN *Staphylococcus Aureus*)

La méthode SLP-HS développée par la société demanderesse est plus sensible. Elle possède des limites de détection (LD) et limites de quantification (LQ) inférieures à la méthode SLP standard :
Test HS SLP LD 1 ng/g et LQ de 3 ng/g.
Test SLP standard LD de 20 ng/g et LQ de 40 ng/g

Ainsi, sauf disposition contraire, le dosage des PGN selon les méthodes classiques est réalisé dans la présente demande à l'aide du test SLP-HS, car, comme il sera exemplifié ci-après et décrit ci dessous, le test SLP-*HS est plus sensible que le test SLP standard.

Les tests de "réponses inflammatoires" tels que décrits ci-dessus, par exemple en utilisant les cellules HEK-Blue™, permettent d'identifier la nature des principaux contaminants (LPS, PGN, β-glucanes, MDP et molécules apparentées) et d'estimer leur part dans l'induction de la réponse inflammatoire.

Les autres contaminants susceptibles d'être présents dans les lots à tester sont essentiellement des glycolipides et lipopeptides agonistes de TLR2 ou des peptides microbiens.
- pour les glycolipides et lipopeptides, une procédure de fractionnement basé sur leur caractère amphiphile est réalisée pour les récupérer et les concentrer.

Un traitement par un mélange chloroforme/méthanol permet d'extraire ces molécules des solutions de polymères de glucose et de les concentrer après évaporation du chloroforme.

Une fois les molécules récupérées, elles sont reprises dans un volume minimum de DMSO (ou tout autre solvant non toxique pour les cellules) puis analysées dans les tests de réponses inflammatoires décrites précédemment.

Ainsi, l'utilisation d'une lignée cellulaire permettant de détecter l'activité d'un récepteur tel que TLR2, par exemple la lignée HEK-Blue™ hTLR2, permet de confirmer ou non la présence de traces d'agonistes de TLR2 autres que le PGN dans les lots à analyser.

Les composés peuvent également être testés dans un modèle utilisant des cellules exprimant tous les types de récepteurs telles que les cellules Raw-Blue™, mais dans ce cas, les solutions de polymères de glucose sont au préalable traitées sur Detoxi-gel, de façon à éliminer les traces de LPS.

En fonction des quantités récupérées, une analyse plus fine des contaminants est réalisée.

Notamment, l'échantillon peut être filtré. Par exemple, l'échantillon de la préparation de polymères de glucose peut être filtré avec un seuil de coupure à 30 kDa, notamment par ultrafiltration. Cela permet d'éliminer les PGN, en particulier les PGN de grande taille. Ainsi, les lipopeptides et les glycopeptides de petite taille, qui sont d'autres agonistes de TLR2, sont conservés dans le filtrat et on peut analyser le filtrat pour déterminer la nature des contaminants.

Par ailleurs, le traitement avec le mélange chloroforme/méthanol permet d'extraire les produits qui sont ensuite fractionnées sur résine C18 et/ou de colonne de charbon. Les composés sont alors élués par un gradient eau/acétonitrile. En fonction de la pureté des fractions et de la quantité de matériel, une analyse plus poussée permet de déterminer la nature biochimique de ces composés, voire leur structure.

Pour les peptides microbiens, une étape d'ultra-filtration sur filtre 5 kDa permet de les extraire des solutions de polymères de glucose. Si nécessaire, un passage sur colonne de charbon permet de débarrasser le filtrat des composés plus hydrophobes de taille < 5 kDa.

Les solutions sont ensuite concentrées puis testées pour leurs propriétés biologiques. Ces peptides étant chimio-attractants pour les leucocytes, leur présence peut être détectée dans un test de migration cellulaire *in vitro* disponible au Laboratoire.

Il se peut que les polymères de glucose soient contaminés par d'autres molécules connues pour déclencher des réponses inflammatoires, telles que la flagelline, une protéine agoniste de TLR5, et les dérivés d'acides nucléiques et molécules apparentées, agonistes des TLR3, 7, 8 et 9 (les trois premiers sont impliqués dans les réactions à des composés d'origine virale, alors que le TLR9 est activé par des ADN d'origine bactérienne).

En ce cas, les lignées cellulaires permettant de détecter l'activité de ces différents TLRs, notamment des lignées HEK-Blue™, sont disponibles et peuvent être utilisées pour analyser l'impact de ces molécules dans les réponses inflammatoires.

De plus, la présence de composés oligonucléotidiques peut être confirmée ou non par des analyses biochimiques.

Le procédé de l'invention permet la détection des contaminants de polymères de glucose destinés à la dialyse péritonéale, contaminants susceptibles d'agir en synergie les uns avec les autres pour déclencher une réaction inflammatoire, caractérisé en ce qu'il comprend au moins un test de réponse inflammatoire *in vitro* à l'aide de lignées cellulaires modifiées.

Dans un mode de réalisation particulier, la présente invention fournit également une méthode de quantification des PGN dans un échantillon, en particulier de polymères de glucose destinés à la dialyse péritonéale, comprenant l'incubation de l'échantillon avec une lignée cellulaire permettant de détecter l'activité du récepteur TLR2 et la mesure de l'activation de la voie de signalisation associée à TLR2, permettant ainsi de déterminer la quantité de PGN contenue dans l'échantillon.

Notamment, cette lignée est une lignée modifiée par transfection (de préférence transfection stable) avec un vecteur codant pour le récepteur TLR2. De préférence, cette lignée n'exprime pas d'autres récepteurs de l'immunité innée. En outre, cette lignée peut contenir un gène rapporteur qui est sous la dépendance directe de la voie de signalisation associée au récepteur TLR2. De manière préférée, ce gène rapporteur code pour une protéine colorée ou fluorescente ou pour une protéine dont l'activité peut être mesurée avec ou sans substrat. De manière particulière, le gène rapporteur code pour une phosphatase alcaline. Ces cellules peuvent par exemple être co-transfectées avec un gène rapporteur produisant, par exemple, une forme sécrétée de la phosphatase alcaline (acronyme anglo-saxon SEAP: *secreted embryonic alkaline phosphatase*), dont la synthèse est sous la dépendance directe de la voie de signalisation associée au récepteur TLR2. Cette lignée peut par exemple être la lignée HEK-Blue™ hTLR2.

Pour déterminer la quantité de PGN contenue dans l'échantillon sur la base de la mesure de l'activation de la voie de signalisation associée à TLR2, une courbe doses-réponses est simultanément établie avec une gamme étalon comprenant des concentrations croissantes de PGN, de préférence de PGN de *S. aureus.*

Préalablement au dosage, l'échantillon à doser peut facultativement avoir été partiellement épuré afin d'éliminer par exemple d'éventuels contaminants gênants. Des glycopeptides et lipopeptides peuvent être éliminés de l'échantillon par extraction chloroformique. Après centrifugation, le dosage sera réalisé sur la phase aqueuse, normalement débarrassée des contaminants à caractère lipophile. Un fractionnement sur microconcentrateur sur filtre de seuils 30 ou 50 kDa peut être réalisé, le dosage étant ensuite réalisé sur le rétentat. Un traitement préalable à la β-glucanase peut permettre d'affiner le dosage en éliminant les molécules apparentées.

D'une manière alternative et préférée, l'échantillon à doser est traité préalablement au dosage par une mutanolysine. Par exemple, l'enzyme à une concentration d'environ 2500 U/ml peut être mise en présence de l'échantillon, préférentiellement dilué pour avoir une concentration en polymère de glucose de 7,5 à 37,5 % (poids/volume) si cela est nécessaire. Le traitement peut durer pendant 6 à 16 h, de préférence pendant environ 16 h. Dans le mode de réalisation préféré, le traitement est réalisé pendant environ 16 h à environ 37°C sur un échantillon présentant une concentration de polymère de glucose d'environ 7,5 % (poids/volume). Ensuite, l'échantillon ainsi traité sera ensuite mise en contact avec des cellules exprimant TLR2, en particulier des cellules HEK-Blue™ hTLR2. Facultativement, le résultat obtenu avec l'échantillon traité par une mutanolysine pourra être comparé au résultat obtenu sans traitement.

Ce même procédé peut être également mis en oeuvre pour la détection des contaminants de polymères de glucose destinés à la nutrition entérale et parentérale, voire même à la nutrition des nouveaux nés.

L'invention sera mieux comprise à l'aide des exemples qui suivent, lesquels se veulent illustratifs et non limitatifs.

### Brève Description des Figures

Figure 1 : Production de RANTES en réponse au PGN et au LPS dans des cellules THP-1 sensibilisées par le MDP à 10 µg/mL.
Figure 2 : Production de TNF-α en réponse au PGN et au LPS dans des cellules THP-1 sensibilisées par le MDP à 10 µg/mL.
Figure 3: Production de RANTES en réponse au PGN dans des cellules THP-1 sensibilisées par le fMLP (10 nM).
Figure 4: Production de RANTES en réponse au PGN dans des cellules THP-1 sensibilisées par le LPS (25 pg/mL).
Figure 5: Effet de la concentration en polymère de glucose sur la production de RANTES induite par le PGN dans les cellules THP-1.
Figure 6 : Effet de la concentration en cellules THP-1 sur la production de RANTES induite par le PGN.
Figure 7 : Production de RANTES par les cellules THP-1 sensibilisées en réponse aux différents échantillons de polymères de glucose.
Figure 8 : Test de réponse des cellules HEK-Blue. Les cellules ont été stimulées par le PGN, le MDP et le TNF-α, qui est un témoin positif de stimulation des cellules HEK-Blue™.
Figure 9 : Réponse SEAP en fonction du volume de surnageant de culture ajouté au milieu réactionnel. L'activation des cellules (HEK-TLR2) a été réalisée avec le TNF-α.
Figure 10 : Optimisation du temps de réaction entre la SEAP et son substrat. Les cellules HEK-TLR2 (Fig 10A) et Raw-Blue (Fig 10B) ont été stimulées en présence de concentrations croissantes de PGN. Après 20 h de stimulation, les surnageants contenant la SEAP ont été incubés en présence de la solution Quanti-blue aux temps indiqués, puis l'absorbance a été mesurée à 620 nm.
Figure 11 : Effet de la concentration en polymère de glucose sur la production de SEAP par les cellules HEK-TLR2 (Fig 11A) et Raw-Blue (Fig 11B) en réponse à des concentrations croissantes de PGN.
Figure 12 : Comparaison des réponses SEAP des cellules HEK-NOD2 cultivées selon la méthode fournie par le fournisseur *versus* la procédure améliorée sans étape de repiquage avant stimulation.
Figure 13 : Production de SEAP en réponse au PGN dans des cellules HEK-TLR2 et HEK-Null.
Figure 14: Production de SEAP en réponse au LTA dans des cellules HEK-TLR2 et HEK-Null.
Figure 15 : Production de SEAP en réponse au PGN et au LPS dans les cellules Raw-Blue.
Figure 16 : Production de SEAP en réponse au MDP dans les cellules HEK-NOD2.
Figure 17 : Activité SEAP dans les cellules HEK-TLR2 en réponse aux différents échantillons de polymères de glucose.
Figure 18 : Activité SEAP dans les cellules Raw-Blue en réponse aux différents échantillons de polymères de glucose.
Figure 19 : Activités SEAP dans les cellules HEK-NOD2 en réponse aux différents échantillons de polymère de glucose.
Figure 20 : Production de SEAP dans les cellules Raw-Blue et HEK-TLR2 en réponse aux différents échantillons de polymère de glucose avant (Total) et après ultrafiltration à 30 kDa (Filtrat).
Figure 21 : Courbe étalon de la réponse cellulaire en fonction du taux de PGN de *S. aureus.* Fig 21A, courbe théorique. Fig 21B, Courbe obtenue avec les cellules HEK-Blue™-hTLR2.
Figure 22 : Activité SEAP dans les cellules HEK-TLR2 en réponse à différents échantillons de polymère de glucose avant et après traitement à la mutanolysine.
Figure 23 : Production de SEAP par les cellules HEK-TLR2 en réponse au PGN avant et après traitement à la mutanolysine.

### Exemple 1 : Préparation des polymères de glucose destinés à la dialyse péritonéale

La matière première pour l'obtention des polymères de glucose selon l'invention est produite à partir d'amidon de maïs waxy de la manière suivante :
- nettoyage du maïs de manière à garder exclusivement les grains de maïs entier,
- trempe du maïs ainsi nettoyé en présence d'acide lactique de manière à assouplir les grains,
- broyage humide, puis séparation des différents constituants, i.e. germe, enveloppe cellulosique, protéines et amidon,
- nettoyage de l'amidon à contre courant avec de l'eau sanitisée de manière à purifier l'amidon aussi bien physico-chimiquement que bactériologiquement,
- centrifugation et séchage de l'amidon,
- mise en suspension de l'amidon dans une eau sanitisée à une matière sèche finale de 40 % et à une Température de 45°C à 50°C,
- acidification de la suspension d'amidon par addition d'HCl à un pH < 2, et accroissement de la température jusqu'à 115 à 120°C pendant 6 à 8 minutes,
- floculation des protéines et des matières grasses à ce pH,
- neutralisation de la suspension à pH 5,
- filtration de la suspension sur terre de diatomées (de manière à retenir les protéines, les matières grasses et la cellulose résiduelles),
- déminéralisation sur résine cationique forte et résine anionique faible,
- traitement au charbon actif à une Tp de 70-80°C et à un pH de 4 à 4,5 ; ce qui élimine les impuretés colorées et réduit le niveau d'impuretés microbiologiques.

Le charbon actif poudre étant ajouté à une concentration comprise entre 0,2 et 0,5% sur sec est retenu sur un filtre céramique de 10 µm chargé auparavant avec un agent filtrant,
- concentration par passage sur évaporateur à film tombant,
- atomisation de la solution concentrée dans un atomiseur de type MSD commercialisée par la société NIRO.

Cet hydrolysat d'amidon est conforme à la monographie de la pharmacopée européenne (réf Maltodextrines : 1542).
∘ pH : 4,0 - 7,0 pour une solution à solution 10 %,
∘ I. d. : conforme,
∘ Perte à la dessiccation : 6 % max
∘ DE : < 20
∘ Cendres sulfuriques : 0,5 % max
∘ SO₂: 20 ppm max
∘ Métaux lourds : < 10 ppm
∘ *E. coli* : absent / g
∘ Salmonelles : absent / 10 g
∘ Germes viables totaux : 100 CFU/g max (EP 1000 CFU/g)
∘ Moisissures : 100 CFU/g max

En complément, sont analysés les lots produits sur les valeurs de :
- contamination en levures + moisissures : 150 CFU/10 g max, soit 15 / g max
- germes aérobies : 500 CFU/10 g max, soit 50/g max
- endotoxines (test LAL gel clot en point final) : 20 EU/g max
- peptidoglycanes : 2700 ng/g max

Les conditions d'obtention des polymères de glucose conformes à l'invention à partir de l'hydrolysat d'amidon ainsi obtenu sont les suivantes :
1) Préparation de l'eau / Qualité de l'eau
   - purification de l'eau par filtration sur 3 µm ; traitement sur charbon actif, déminéralisation sur résines échangeuses de cations et d'anions, et filtration à nouveau (UA),
   - deux réservoirs utilisés :
      ∘ 10 m³ pour la dissolution de l'hydrolysat d'amidon, les étapes de rinçage et nettoyage de l'atomiseur,
      ∘ 60 m³ pour le procédé principal (nettoyage des réservoirs, ses suspensions de charbon actif et de la chromatographie
3) Chromatographie
   - solubilisation de l'hydrolysat d'amidon avec de l'eau purifiée de manière à obtenir une MS de 35 - 45 % à une température comprise entre 60 - 85°C,
   - filtration stérilisante de l'hydrolysat d'amidon par passage sur 0,45 µm puis 0,22 µm, réalisée à un ΔP < 3 bars,
   - séparation chromatographique par exclusion stérique (SEC) réalisée à l'aide d'un système continu composé de 6 séries de double plateaux de 1 m³ de résine chacun. La résine mise en oeuvre est une PCR145K commercialisée par la société Purolite.

La solution qui traverse cette résine présente une température comprise entre 75 et 85°C à 35-45 % de MS.

La durée de chaque séquence définit le procédé.

Dans le cas présent, la durée de chaque séquence est de 15 minutes.

Le contrôle est effectué par une analyse de distribution du poids moléculaire et l'analyse du rendement de chromatographie, de la manière suivante : (Quantité de matière sèche de la fraction voulue)/ (Quantité de matière sèche de l'alimentation)

Les poids moléculaires les plus faibles interagissent avec la résine et les haut poids moléculaires sont élués à l'eau purifiée.

La concentration est effectuée par évaporation en film tombant à une MS de 35 - 45 %.

On réalise un traitement thermique à une température de 120°C pendant 2 minutes,

On ajoute le charbon actif entre 0,5 et 1,5 % de la masse totale de l'hydrolysat d'amidon à 75°C avec des résines cationiques (1 à 3 l) pour contrôler le pH (4 - 4,5) et anioniques (5 à 10 l) pour contrôler le pH (5,5 - 6),

On filtre sur filtres à manches en polypropylène avec ΔP < 5 bars, en 5 à 6 heures par lot.

On effectue une seconde et une troisième filtration sur 1,5 et 0,45 µm, puis sur 0,22 et 0,1 µm, et une ultrafiltration sur membrane d'un seuil de coupure de l'ordre de 40.000 Da

Pour l'atomisation : alimentation à 500 kgs/h avec une solution à 40% de MS et à 250°C dans un atomiseur de type MSD commercialisé par la société Niro.

Le produit atomisé présente à sa sortie une humidité inférieure à 6 %.

On refroidit le produit alors en lit d'air fluidisé comprenant 3 zones de refroidissement alimentée par de l'air à 40, 30 et 20°C. Le produit obtenu est ensuite tamiser sur 800µm afin de retirer les agrégats.

De l'ordre de 500 kgs de produit fini sont obtenus à partir de 800 kgs de maltodextrines départ, soit un rendement de l'ordre de 60%.

La détermination de la contamination éventuelle du circuit est réalisée par l'analyse de la teneur en peptidoglycanes et endotoxines sur le produit fini.

Pour exemple, les teneurs habituellement observées et mesurées sur les lots du produit fini (exprimés par g de polymère de glucose) sont pour les critères spécifiés ci-dessus les suivants :
- Levures et moisissures : 0 / g
- Germes aérobies : 0/g
- Endotoxines (test LAL gel clot en point final) : ≤ à 0,3 EU/g.
- Peptidoglycanes : < 3 ng/g
- *B. acidocaldarius* : 1/g

### Exemple 2 : Utilisation de la lignée cellulaire THP-1 "sensibilisées" pour la détection de contaminants pro-inflammatoires

### Matériels & Méthodes

Les cellules THP-1 (88081201, ECACC) sont cultivées en routine au Laboratoire.

Pour les expériences d'activation pro-inflammatoire, les cellules THP-1 sont différenciées pendant 3 jours en présence de phorbol ester (PMA). En particulier, les cellules sont mises en culture dans 200 µl de milieu complet en présence de 20 nM de PMA pendant 72 h (densité cellulaire finale : 0,75 10⁶ cellules/mL).

Les échantillons de polymères de glucose sont préparés selon l'exemple 1.

**Tableau 1 : Echantillons de polymères de glucose**

| | I-10.01 | I-10.02 | I-10.03 | I-11.12 | MM-10.04 | MM-10.05 | MP-10.06 | MP-10.07 |
|---|---|---|---|---|---|---|---|---|
| Test LAL (EU/g) | < 0,3 | 0,3 | < 0,3 | 0,6 | 1,2 | 9,6 | < 0,3 | 38,4 |
| Test SLP standard (ng/g) | < 20 | 755 | 27 | 530 | < 20 | 2755 | < 20 | 4613 |
| Test SLP-HS (ng/g) | < 3 | 253 | 12 | 393 | < 3 | 501 | < 3 | 645 |

Les molécules standards pour l'établissement des gammes étalons sont pour les:
- Test LAL : LPS d'*E*.*col*i O55B5
- Test SLP Standard : PGN de *M. luteus* - WAKO
- Test SLP-HS : PGN de *S. aureus* - WAKO

Les valeurs dosées de PGN diffèrent d'un test WAKO à l'autre du fait de la réactivité et sensibilité différente de ces tests et potentiellement de leur spécificité limitée et relative (en particulier, réponse possible aux β-glucanes).

Les études d'optimisation sont réalisées avec des solutions de polymères de glucose I-10.01 (tableau 1) chargées artificiellement en molécules inflammatoires standards : PGN et MDP (source : *S. aureus*), LPS (source : *E. coli*), f-MLP (peptide de synthèse).

La solution de dilution des standards est I-10-01 avec < 0,3 EU/g de LPS (Test LAL), < 20 ng/g de PGN (Test SLP standard) et < 3 ng/g (Test SLP-HS) et utilisée à la concentration de 5 mg/mL finale.

Les analyses sont ensuite effectuées sur une première série d'échantillons correspondant à différents lots sélectionnés sur la base des taux de contamination des impuretés mesurées par les tests LAL et SLP (PGN, LPS et β-glucanes).

Les kits ELISA de dosage de TNF-α et de CCL5/RANTES sont achetés chez AbCys, les agonistes standards (PGN, LPS, f-MLP et MDP) chez Sigma Aldrich et InvivoGen.

Les cellules THP-1 différenciées (0,75.10⁶ cellules/mL) sont mises en culture dans 200 µl de milieu complet, puis incubées en présence des différents échantillons à tester.

Chaque analyse est réalisée en triplicate.

Les surnageants cellulaires sont collectés afin de doser les cytokines sécrétées après 8 h de stimulation pour le TNF-α, et 20 h pour RANTES.

Les dosages ELISA sont réalisés selon les indications données par le fournisseur.

### Résultats

Les premiers essais ont consisté à tester le potentiel synergique du MDP et du f-MLP, ainsi que la combinaison PGN/LPS, sur la production de cytokines pro-inflammatoires par les cellules THP-1 différenciées.

Le MDP a été testé aux doses de 1, 10 et 100 µg/mL. La dose minimale n'induit pas d'effet synergique significatif. Par contre, les doses de 10 et 100 µg/mL ont une activité synergique similaire sur la production de RANTES et de TNF-α en réponse aux PGN et LPS.

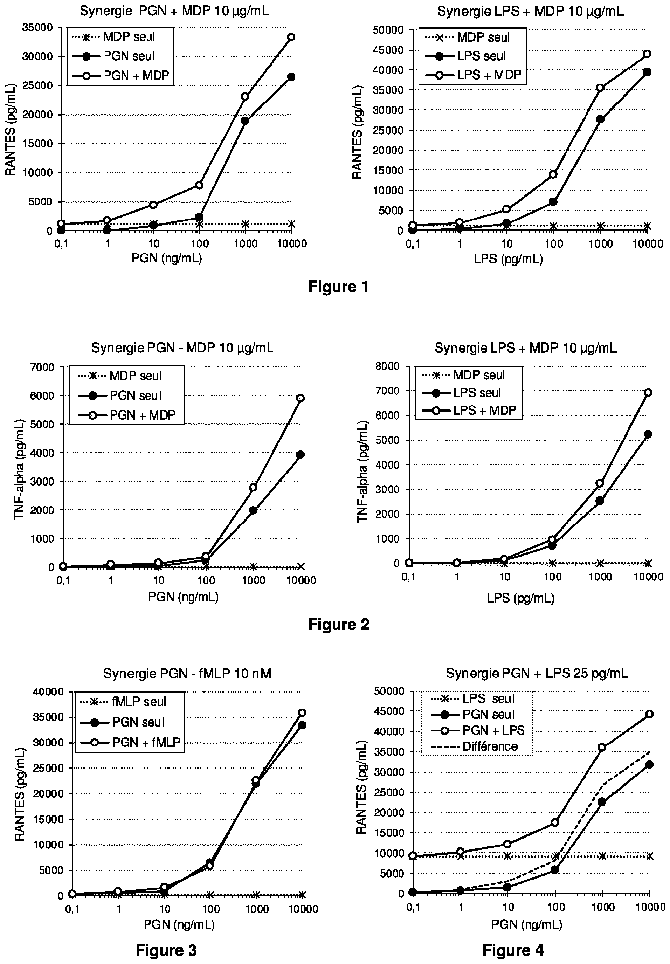

Les résultats présentés dans les Figures 1 et 2 montrent clairement un effet synergique du MDP sur la production de RANTES. En revanche, cet effet synergique est peu marqué pour le TNF-α. De plus, le seuil de détection (taux de PGN ou de LPS donnant une réponse supérieure à 3 fois le SD du bruit de fond) est plus bas pour RANTES (voir Tableau 2).

Le f-MLP a été utilisé aux doses de 1, 10 nM et 100 nM. Toutefois, aucun effet synergique n'a été observé dans les cellules THP-1 (Figure 3).

L'effet synergique du LPS a été analysé en ajoutant une dose sub-optimale (25 pg/mL) à des doses croissantes de PGN (Figure 4). L'effet synergique entre les deux agonistes est visible après dosage des deux cytokines. Toutefois, l'effet synergique induit par le LPS sur la production de RANTES reste inférieur à celui du MDP.

La réponse a été quantifiée en mesurant la production de RANTES et de TNF-α. Dans tous les cas, la synergie est nettement visible pour le dosage de RANTES, avec une sensibilité élevée. Ce dosage sera donc privilégié et conservé pour la suite des expérimentations.

Ces résultats montrent que l'addition de MDP à 10 µg/mL aux cellules THP-1, avec dosage en retour de RANTES, est le mode de sensibilisation le plus efficace pour détecter de faibles taux de PGN et de LPS. En théorie, ces seuils de détection doivent permettre de détecter les contaminants présents dans les produits manufacturés.

Ces premières analyses ont été réalisées en diluant les molécules inflammatoires standards en présence du polymère I-10.01. Les solutions ont été ajoutées aux cellules THP-1 de façon à obtenir une concentration finale en polymère de glucose de 5 mg/mL et une densité cellulaire finale de 0,75.10⁶ cellules/mL. Afin d'augmenter la sensibilité du dosage, les essais ont été réalisés en faisant varier ces deux paramètres.

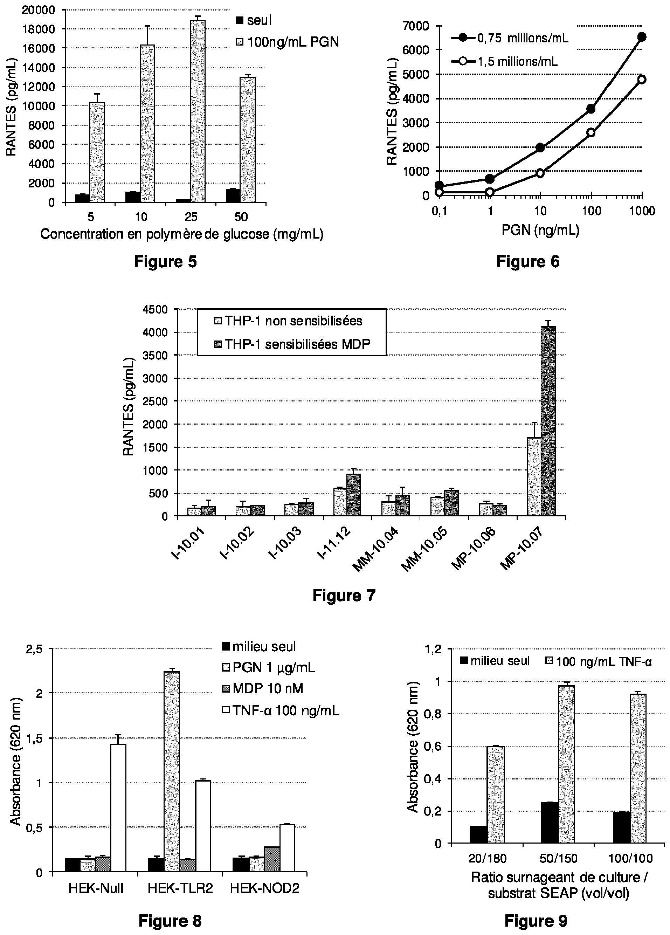

La présence du polymère de glucose ne gêne pas la production de RANTES pour des concentrations inférieures ou égales à 25 mg/mL. Cette augmentation de la sensibilité n'est pas liée à un effet pro-inflammatoire du polymère sur les cellules, puisque la réponse est identique au bruit de fond en absence de PGN (Figure 5).

Par contre, l'augmentation de la densité cellulaire au-delà de 0,75.10⁶/mL diminue la production de RANTES en réponse au PGN (épuisement du milieu de culture ou altération cellulaire) (Figure 6).

Les tests de sensibilisation avec le MDP à 10 µg/mL ont été reproduits 3 fois pour le LPS et 6 fois pour le PGN avec des cellules THP-1 provenant de préparations distinctes. Les données obtenues ont permis de déterminer les seuils de détection et les EC50 (Tableau 2). Pour l'estimation de la sensibilité, les valeurs ont été ramenées par g de polymère en considérant la concentration de 25 mg/mL.

L'effet synergique induit par le MDP est identique pour le LPS et le PGN, puisqu'il permet d'augmenter la sensibilité des cellules THP-1 d'un facteur 5 dans les deux cas.

**Tableau 2 : Seuils de détection et EC50 pour le PGN et le LPS dans le modèle THP-1 sensibilisées.**

| | PGN | PGN + MDP | LPS | LPS + MDP |
|---|---|---|---|---|
| Seuil de détection | 14,5 ± 8,5 ng/mL | 2,8 ± 1,2 ng/mL | 10 ± 7 pg/mL | 2 ± 1 pg/mL |
| EC50 | 1,2 ± 0,7 µg/mL | 0,4 ± 0,2 µg/mL | 0,9 ± 0,1 ng/mL | 0,3 ± 0,1 ng/mL |
| Sensibilité | 580 ± 340 ng/g | 112 ± 48 ng/g | 400 ± 280 pg/g | 80 ± 40 pg/g |

Ces travaux montrent que les cellules THP-1 sensibilisées peuvent être utilisées pour développer un test de réponse inflammatoire sensible pour détecter les traces de contaminants dans les préparations de polymère de glucose.

Les conditions expérimentales suivantes sont retenues :
- concentration finale des cellules THP-1 différenciées : 0,75.10⁶ cellules/mL.
- agent de sensibilisation : MDP (*S. aureus*) à 10 µg/mL.
- concentration finale des polymères de glucose : 25 mg/mL.
- réponse : dosage ELISA de la production de RANTES après 20 h de stimulation.

Afin de valider la méthode, les essais avec les cellules THP-1 sensibilisées par le MDP ont été réalisés avec les échantillons présentés dans le tableau 1.

Le test basé sur la production de RANTES par les cellules THP-1 sensibilisées permet de détecter les échantillons de polymères contaminés par du LPS : polymères référencés I-11.12, MP-10.07, et dans une moindre mesure MM-10.04 et MM-10.05 (Figure 7).

Par contre, les échantillons contaminés uniquement par du PGN (e.g. I 10-02) ne donnent pas de réponse, ce qui indique que ce test cellulaire est plus adapté pour la détection des endotoxines. Toutefois, l'amplitude des réponses n'est pas directement proportionnelle au taux de LPS mesuré par le test LAL (voir par exemple les réponses obtenus avec I-11.12 versus MM-10.05), ce qui suggère que des contaminants autres que le PGN agissent probablement avec le LPS sur la réponse des cellules THP-1.

Les cellules THP-1 répondent aux solutions de PGN témoin, mais pas ou peu aux échantillons provenant de lots contaminés uniquement par le PGN naturel.

La taille des PGN est très hétérogène, et certaines de ces molécules peuvent atteindre des masses imposantes (> 10⁶ Da). Ces derniers sont peu solubles, ce qui affecte leur pouvoir pro-inflammatoire mais favorise leur élimination par filtration. Par contre, les PGN solubles, et par conséquent actifs, ont une masse moyenne de 120 kDa et ne sont pas éliminés par filtration. Il est donc envisageable que les deux tests SLP de Wako permettent un dosage global de tous les PGN, alors que le test cellulaire ne détecte que les PGN actifs.

### Exemple 3 : Utilisation des lignées cellulaires HEK-Blue™ (hTLR2, hNOD2, Null2) et Raw-Blue™ (InvivoGen) pour la détection de contaminants

### Matériels & Méthodes

Les lignées cellulaires HEK-Blue™ (InvivoGen) sont des lignées modifiées par transfection stable avec des vecteurs codant pour des récepteurs de l'immunité innée. Ces cellules sont également co-transfectées avec un gène rapporteur produisant une forme sécrétée de la phosphatase alcaline (SEAP: *secreted embryonic alkaline phosphatase*), dont la synthèse est sous la dépendance directe de la voie de signalisation associée au(x) récepteur(s) exprimé(s) dans la même lignée cellulaire.

Pour les expériences relatives à la détection des contaminants inflammatoires, quatre lignées sont utilisées :
- lignée HEK-Blue™ hTLR2 (HEK-TLR2) : cette lignée répond spécifiquement aux agonistes du TLR2 (notamment PGN et la majorité des glycolipides et lipopeptides).
- lignée HEK-Blue™ hNOD2 (HEK-NOD2) : cette lignée répond au MDP et molécules apparentées, telles que les PGN monomériques.
- lignée HEK-Blue™ Null2 (HEK-Null) : il s'agit d'une lignée contrôle, dont l'utilisation est nécessaire pour vérifier que les solutions de polymères de glucose n'induisent pas la production de l'enzyme par un mécanisme intrinsèque.
- lignée Raw-Blue™ : il s'agit d'une lignée de macrophages de souris transfectée par la SEAP. Cette lignée qui exprime de façon naturelle la quasi-totalité des récepteurs de l'immunité innée est utilisée comme témoin positif dans les tests.

Les cellules Raw-Blue™ et HEK-Blue™ sont cultivées selon les recommandations du fournisseur. En particulier, la pression de sélection des plasmides codant pour les récepteurs des molécules inflammatoires (TLR2 ou NOD2) et pour la SEAP est assurée en ajoutant dans le milieu de culture les antibiotiques HEK-Blue Sélection/blasticidine. A 75% de confluence, les cellules sont remises en suspension à une densité de 0,28.10⁶ cellules/mL. Avant stimulation, 180 µL de la suspension cellulaire sont répartis dans les puits de culture (boite de 96 puits), soit 50.000 cellules/puits. Les cellules sont ensuite stimulées pendant 24 h par addition de 20 µL des échantillons à tester (sous forme 10 fois concentrée). La stimulation dure de 16 à 24 h.

La production de SEAP en réponse aux molécules contaminants est estimée en mesurant l'activité de la phosphatase selon le protocole fourni par le fabricant : 20 µL de surnageant de culture sont dilués dans 180 µL de Quanti-Blue. Le développement de la coloration se fait à 37°C et la lecture est réalisée à différents intervalles à 620 nm. Les données sont exprimées en absorbance après soustraction du bruit de fond, obtenu en ajoutant le même volume de milieu de culture non conditionnée au milieu réactionnel de l'enzyme.

Les études d'optimisation sont réalisées avec des solutions de polymère de glucose I-10.01 (tableau 1) chargées artificiellement en molécules inflammatoires standards : PGN, LTA et MDP (source : *S. aureus*), LPS (source : *E.coli*). Les analyses sont ensuite effectuées sur la série d'échantillons correspondant à différents lots sélectionnés sur la base des taux de contamination par le PGN et le LPS (Tableau 1).

### Résultats

Les cellules HEK-Blue sont réceptionnées sous formes d'ampoules congelées. Avant de débuter les tests de réponse inflammatoire, il faut s'assurer de la reprise des cellules et de leur capacité à répondre aux molécules inflammatoires. De plus, ces cellules transfectées dégénèrent rapidement après plusieurs passages, ce qui peut se traduire par une perte des vecteurs d'expression pour les récepteurs de l'immunité innée, voire du vecteur codant pour la SEAP.

Il est donc préconisé de vérifier la capacité des cellules à répondre aux facteurs inflammatoires au début de mise en culture, puis après plusieurs étapes de repiquage. Ces tests sont réalisés avec le PGN pour HEK-TLR2, le MDP pour HEK-NOD2 et le TNF-α, ce dernier étant un puissant activateur de la voie NF-κB. En effet, les cellules HEK possèdent de façon naturelle le récepteur de cette cytokine, et vont donc produire la SEAP (dont le vecteur d'expression est sous la dépendance de la voie NF-κB) en réponse au TNF-α.

Les résultats présentés en Figure 8 montrent les tests réalisés pour vérifier la reprise des trois lignées. Comme attendu, les cellules HEK-TLR2 et HEK-Null répondent au TNF-α, avec une production de SEAP équivalente. De plus, la lignée HEK-Null est insensible au PGN et au MDP, alors que la cellule HEK-TLR2 ne répond efficacement qu'au PGN. Ces deux lignées ont donc acquis leurs caractéristiques phénotypiques et vont pouvoir être utilisées pour la suite des expérimentations. Dans cet exemple, la lignée HEK-NOD2 ne répond que très faiblement au TNF-α et au MDP, ce qui indique qu'elle n'a pas acquis les caractéristiques attendues.

La réponse des cellules HEK-Blue et Raw-Blue aux molécules pro-inflammatoires est directement liée à la production de la SEAP. Le fournisseur préconise d'ajouter 20 µL de surnageant de culture à 180 µL de substrat de la SEAP. Les expériences ont donc été réalisées dans ces conditions, puis optimisées en augmentant le volume de surnageant de culture, et donc la quantité d'enzyme en solution (Figure 9).

Les résultats montrent que le ratio 50/150 donne une réponse plus élevée que le ratio préconisé par le fournisseur. Par contre, le ratio 100/100 n'est pas plus efficace, probablement par manque de substrat de la SEAP dans le milieu réactionnel.

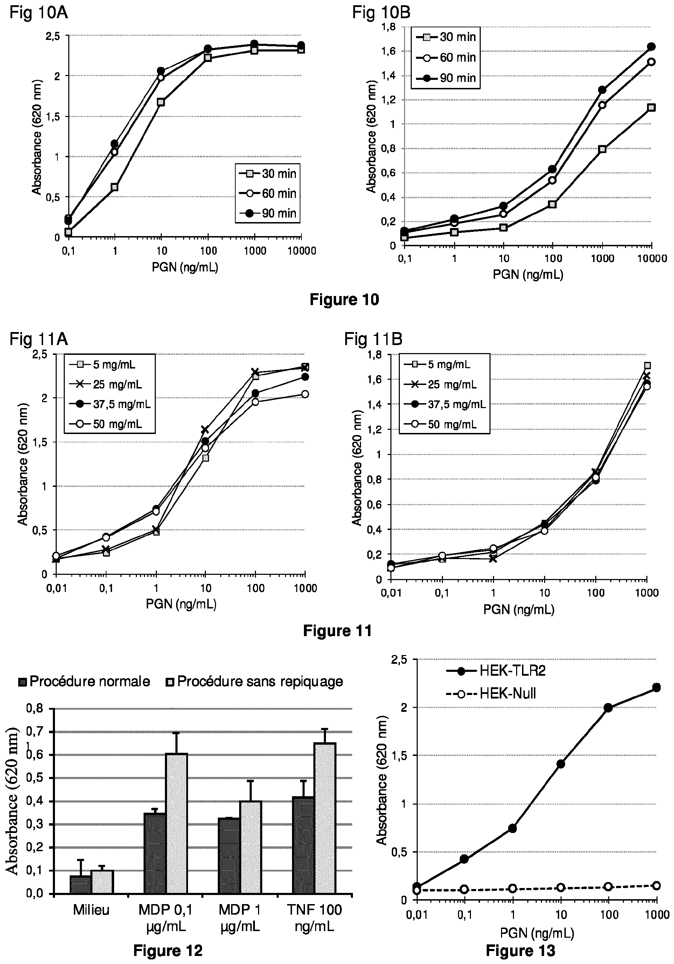

L'intensité de la coloration (620 nm) est proportionnelle à la quantité de SEAP sécrétée par les cellules, mais également au temps d'hydrolyse du substrat par l'enzyme. Différents temps de révélation ont donc été testés à partir des mêmes surnageants des cellules HEK-TLR2 et Raw-Blue activées par le PGN (Figure 10).

La coloration optimale est atteinte à partir de 60 min de réaction entre la SEAP et son substrat pour les cellules HEK-TLR2. On observe encore une légère augmentation à 90 min pour les cellules Raw-Blue, mais qui est probablement liée au fait que ces cellules produisent moins de SEAP.

L'effet de la concentration en polymère de glucose sur les réponses des cellules a été analysé en stimulant les cellules HEK-TLR2 et Raw-Blue par du PGN standard dilué dans du milieu additionné du polymère I-10.01. Les solutions ont ensuite été ajoutées aux cellules de façon à obtenir des concentrations finales en polymère variant de 5 à 50 mg/mL (Figure 11).

Les concentrations jusqu'à 37,5 mg/mL ne modifient pas de façon significative l'amplitude de la réponse au PGN dans la lignée HEK-TLR2. On note même une amélioration de la réponse aux faibles concentrations de PGN pour des concentrations de polymères supérieures à 25 mg/mL, probablement liée à une meilleure dispersion du contaminant. Quant aux cellules Raw-Blue, la production de SEAP n'est pas modifiée quel que soit la concentration en polymère de glucose.

La lignée HEK-NOD2 montre une amplitude de réponse faible au MDP et même au TNF-α, ce qui apparaît être lié à un important bruit de fond. Dans ces cellules, le gène de la SEAP est sous la dépendance d'un promoteur faible, plus sensible au stress cellulaire que celui utilisé pour les autres cellules HEK-Blue. Afin de diminuer le bruit de fond et augmenter l'amplitude de la réponse aux contaminants, les conditions de culture ont été modifiées, de façon à diminuer le stress des cellules.

Selon les recommandations du fournisseur, les cellules à 75% de confluence sont remises en suspension à une densité de 0,28.10⁶ cellules/mL, puis 180 µL de la suspension cellulaire sont repartis dans les puits de culture (boite de 96 puits), soit 50.000 cellules/puits, avant stimulation dans la journée.

Dans la nouvelle procédure dite sans repiquage, les cellules HEK-NOD2 sont conditionnées en puits (10.000 /puits) et cultivées pendant trois jours. Avant stimulation, le milieu de culture est enlevé et remplacé par un milieu contenant 1% de SVF et les solutions à doser. Dans ce cas, la réponse SEAP est 5-6 fois supérieure au témoin négatif, ce qui est compatible avec un test de détection du MDP dans des solutions contaminées (Figure 12).

Ces premiers travaux montrent que les cellules HEK-Blue et Raw-Blue peuvent être utilisées pour développer des tests de réponse inflammatoire sensibles pour détecter les traces de contaminants dans les préparations de polymère de glucose.

Les conditions expérimentales suivantes ont été retenues :
- concentration finale du polymère de glucose : 37,5 mg/mL pour les cellules HEK-Blue, et 50 mg/mL pour les cellules Raw-Blue.
- réaction enzymatique : 50 µL de milieu conditionné + 150 µL de réactif SEAP.
- temps de réaction minimum : 60 min.

Les Figures 13-16 montrent des exemples de dosages de l'activité SEAP produite par les cellules HEK-TLR2 et Raw-Blue en réponse à différents molécules inflammatoires.

Les cellules HEK-TLR2 répondent très efficacement au PGN, alors que la réponse au LTA est plus faible. Les réponses sont toutefois plus efficaces que celles observées avec des cellules de type monocyte/macrophage. En outre, le polymère de glucose n'a pas d'effet direct sur la production de SEAP, puisqu'aucune réponse n'est observée dans les surnageants de cellules HEK-Null.

Les cellules Raw-Blue répondent bien au PGN, mais sont moins sensibles que les cellules HEK-TLR2. La réponse au LPS est faible et reste bien inférieure à celle observée en mesurant la production de RANTES par les cellules THP-1.

Contrairement aux autres lignées cellulaires, les cellules HEK-NOD2 répondent efficacement au MDP, ce qui peut être mis à profit pour détecter les produits de dégradation des PGN.

Les expériences avec les PGN, le LPS et le MDP standards ont été reproduites avec des préparations cellulaires différentes, ce qui a permis de déterminer les caractéristiques présentées dans le tableau 3. Pour l'estimation de la sensibilité, les valeurs ont été ramenées par g de polymère en considérant la concentration de 37,5 mg/mL pour les cellules HEK-Blue et 50 mg/mL pour les cellules Raw-Blue.

**Tableau 3 : Seuils de détection et EC50 pour le PGN, le LPS et le MDP dans les modèles HEK-Blue et Raw-Blue.**

| | Lignée HEK-TLR2 | Lignée HEK-NOD2 | Lignée Raw-Blue | |
|---|---|---|---|---|
| | PGN | MDP | PGN | LPS |
| Seuil de détection | 0,07 ± 0,04 ng/mL | 1,5 ± 0,5 ng/mL | 2,1 ± 1,5 ng/mL | 0.24 ± 0,06 ng/mL |
| EC50 | 3,1 ± 2,5 ng/mL | 12 ± 6 ng/mL | 210 ± 75 ng/mL | > 10 ng/mL |
| Sensibilité | 1,9 ± 1,1 ng/g | 40 ± 13 ng/g | 42 ± 30 ng/g | 4,8 ± 1,2 ng/g |

Les lignées HEK-TLR2 et Raw-Blue sont très efficaces pour détecter les PGN à des concentrations faibles. En particulier, la lignée HEK-TLR2 détecte des taux de PGN inférieurs à 2 ng/g de polymère de glucose, soit un seuil de sensibilité 50 fois supérieur à celui obtenu avec les cellules THP-1 sensibilisées. La lignée Raw-Blue détecte efficacement de faibles traces de PGN, mais elle est peu réactive vis-à-vis du LPS, avec un seuil de détection environ 50 fois supérieur à celui des cellules THP-1 sensibilisées.

Pour valider ces tests, les essais ont donc été réalisés avec des échantillons de polymères de glucose.

La lignée HEK-TLR2 permet de détecter des contaminations dans les lots I-10.02, I-11.12, MM-10.05, et dans une moindre mesure, MP-10.06 et MP-10.07 (la réponse observée avec MM-10.04 n'est pas significative en comparaison avec I-10.01 et n'est donc pas retenue).

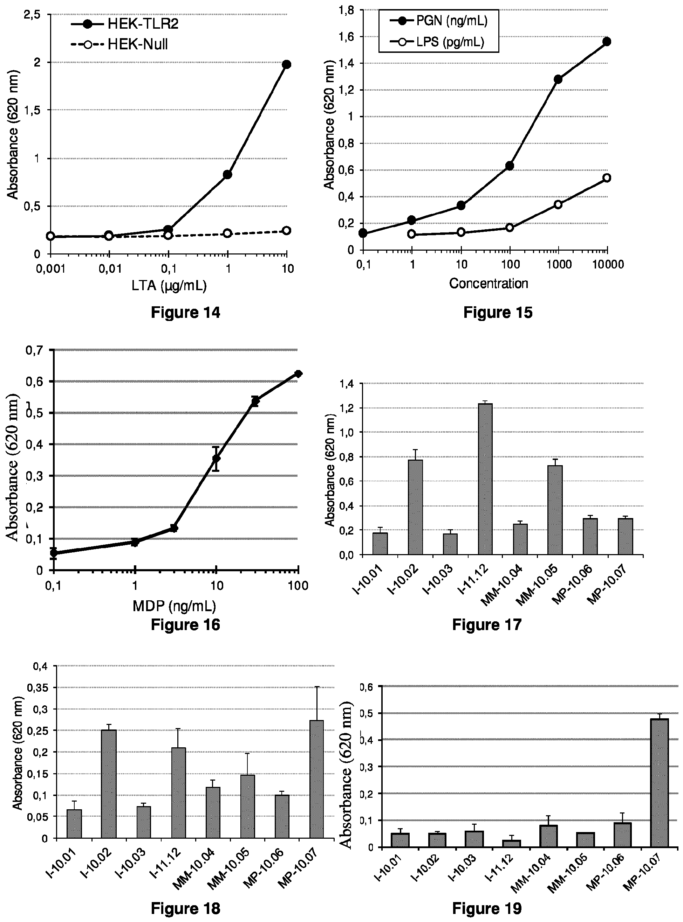

Par contre, l'échantillon I-10.03 n'est pas détecté, ce qui peut être corrélé au taux de PGN très faible, proche de la limite de détection du test SLP-Wako (Figure 17).

Si les réponses positives obtenues avec I-10.02, I-11.12, MM-10.05 et MP-10.07 étaient attendus, étant donné le taux élevé de PGN présent dans ces 4 échantillons, on peut noter qu'il n'y a pas de proportionnalité entre la concentration donnée par le test SLP et la réponse des cellules.

En effet, les polymères de glucose I-10.02, I-11.12 donnent les réponses les plus élevées, alors que les taux de PGN sont inférieurs à 1 µg/g. A l'inverse, l'échantillon MP-10.07, qui est le plus chargé en PGN, donne une réponse proche du bruit de fond. Les PGN sont des macromolécules de masse très variable, et il a été démontré que leur réactivité est inversement proportionnelle à leur masse moléculaire. Il est donc envisageable que les PGN des I-10.02, I-11.12 soient de taille inférieure à celle des PGN présents dans les échantillons MM-10.05 et MP-10.07, ce qui expliquerait leur potentiel pro-inflammatoire plus élevé.

Il est également surprenant de voir une réponse avec le lot MP 10-06, alors qu'il ne contient pas de PGN. Toutefois, les cellules HEK-TLR2 réagissent avec d'autres molécules inflammatoires, telles que les lipopeptides, les LTA, les LAM (lipoarabinomannane), tous agonistes du TLR2. Ainsi, ce résultat suggère que cet échantillon non contaminé en termes d'absence de LPS et de PGN contient d'autres molécules pro-inflammatoires agonistes de TLR2.

A l'exception des échantillons I-10.01 et I-10.03, tous les échantillons donnent une réponse positive avec les cellules Raw-Blue (Figure 18).

Le taux de LPS présent dans l'échantillon I-10-02 est proche du seuil de détection du test LAL, ce qui indique que la réponse observée est due à la présence de PGN. Ce résultat confirme que, contrairement aux cellules THP-1 qui ne répondent à cet échantillon, les cellules Raw-Blue sont efficaces pour détecter de faibles contaminations par le PGN. La forte réponse des lots I-11.12, MM-10.05 et MP-10.07 serait donc due à la présence des deux contaminants (PGN et LPS).

Comme les cellules HEK-TLR2, les cellules Raw-Blue répondent positivement à MP 10-06, ce qui confirme la présence d'un contaminant autre que le LPS et les PGN dans cet échantillon.

Finalement, les cellules HEK-NOD2 réagissent fortement en présence de MP-10.07, et donnent une réponse faible mais significative avec les échantillons I-10.03, MM-10.04 et MP-10.06, ce qui indique que ces échantillons ont été contaminés par des PGN à une étape de leur fabrication, et que ces derniers ont été partiellement dégradés en cours de fabrication du produit (Figure 19).

Les cellules HEK-TLR2 et Raw-Blue sont efficaces pour détecter la trace de contaminants inflammatoires dans des produits de type I-10.01 et I-10.03.

Elles ont même des caractéristiques complémentaires aux cellules THP-1 sensibilisées. En effet, les tests utilisant les trois permettent de détecter les la majorité des contaminants inflammatoires susceptibles d'être présents dans des échantillons de polymères de glucose.
- THP-1 : tout contaminant inflammatoire avec une réactivité élevée pour le LPS.
- Raw-Blue : tout contaminant inflammatoire avec une réactivité élevée pour les PGN.
- HEK-TLR2 : spécifique des agonistes TLR2 avec une forte réactivité pour les PGN.
- HEK-NOD2 : spécifique du MDP et par conséquent des produits de dégradation des PGN.Comme pour les cellules THP-1, on note encore une absence de proportionnalité entre les taux de PGN et/ou de LPS et les amplitudes des réponses cellulaires.

Ce problème est certainement lié à la taille de ces macromolécules et/ou à leur présence sous forme d'agrégats, ce qui influence leur réactivité vis-à-vis des cellules. Ainsi, nous avons observé que le passage sur filtre 0,22 µm réduit d'environ 50 % la réactivité du PGN standard pour les cellules HEK-TLR2. C'est pourquoi dans tous les tests, les solutions de polymères de glucose sont préparées en conditions aseptiques mais sans étape de filtration des molécules standards.

### Exemple 4 : Caractérisation des contaminants par l'utilisation des lignées cellulaires THP-1 sensibilisées, HEK-Blue™ (hTLR2, hNOD2) et Raw-Blue™.

Les exemples 2 et 3 montrent que les lignées THP-1 sensibilisées, HEK-TLR2, HEK-NOD2 et Raw-Blue sont efficaces pour détecter la trace de contaminants inflammatoires dans les échantillons de polymères de glucose. Outre le LPS et le MDP dont la présence est détectée *via* les récepteurs TLR4 et NOD2, les autres contaminants susceptibles d'être présents dans les échantillons sont majoritairement des ligands de TLR2 (PGN, LTA et lipopeptides). Par conséquent, les lignées ne permettent pas d'établir la part des PGN dans la réponse TLR2-spécifique. Toutefois, les PGN sont des macromolécules dont la masse varie de ∼ 100 kDa à plusieurs millions de Da. A l'inverse, les LTA et lipopeptides ont des masses faibles, inférieures à 15 kDa. Ainsi, l'introduction d'une étape d'ultrafiltration (30 kDa) doit permettre de retenir les PGN et de ne mesurer que la réponse aux ligands de TLR2 de petites tailles.

### Procédures expérimentales

Pour les expériences relatives à cet exemple, les cinq lignées cellulaires présentées dans les exemples 2 et 3 sont utilisées :
- lignée monocytaires THP-1 : réponse à tout contaminant inflammatoire avec une réactivité élevée pour le LPS.
- lignée Raw- Blue™: réponse à tout contaminant inflammatoire avec une réactivité élevée pour les PGN.
- lignée HEK-Blue™ hTLR2 : spécifique des agonistes TLR2 avec une forte réactivité pour les PGN.
- lignée HEK-Blue™ hNOD2 : spécifique des produits de dépolymérisation totale des PGN (MDP).
- lignée HEK-Blue™ Null: témoin de réponse négative.

Les échantillons de polymères de glucose sont présentés dans l'exemple 2 (Tableau 1). Ces échantillons sont préparés en solution aux concentrations décrites dans les exemples 2 et 3. Les réponses cellulaires (production de RANTES pour les cellules THP-1 et sécrétion de la SEAP pour les cellules Blue™) sont analysées soit avec les échantillons non filtrés : Réponse Totale, soit avec les filtrats obtenus par ultra-filtration sur microconcentrateur avec un seuil de coupure à 30 kDa (Sartorius) : Réponse Filtrat.

Avant utilisation, les micro-concentrateurs ont été traités avec une solution saline (NaCl 150 mM) préparée avec de l'eau apyrogène. Les rétentats et filtrats ont été testés avec les lignées cellulaires, et seuls les filtres donnant une réponse négative dans chaque test ont été retenus pour les analyses avec les échantillons de polymères de glucose.

### Résultats

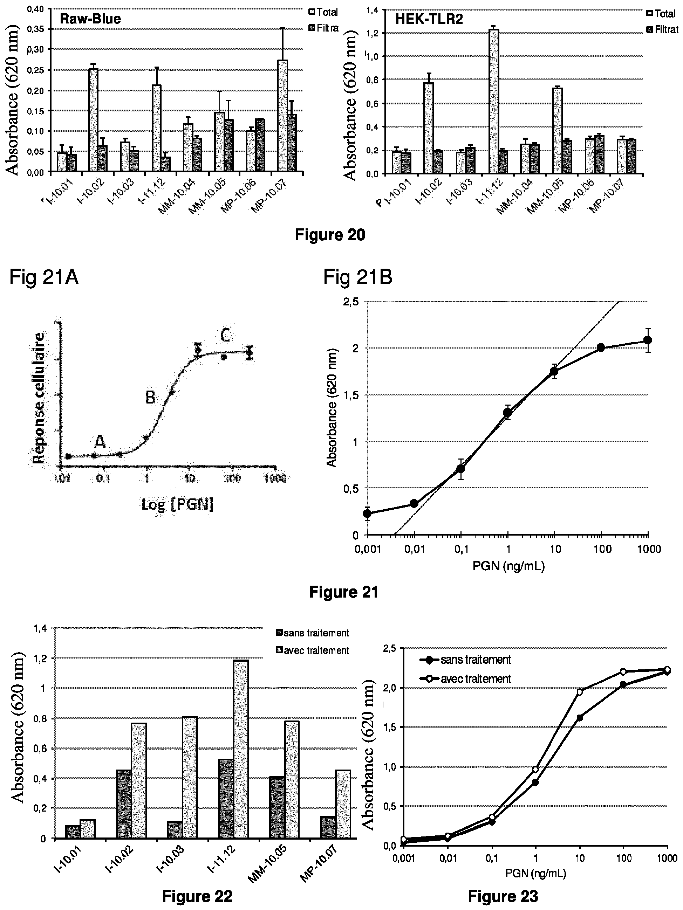

Les résultats présentés dans la Figure 20 montrent les réponses des cellules HEK-TLR2 et Raw-Blue obtenues en présence des différents échantillons avant et après ultra-filtration.

Les réponses Totales sont similaires à celles observées dans l'exemple 3 (Figures 17 et 18).

Les réponses Filtrats sont fortement réduites pour les échantillons I-10.02 et I-11.12 dans les deux types cellulaires, avec des valeurs proches ou égales aux seuils de détection. Ces données confirment que ces deux échantillons sont contaminés par du PGN, qui a été retenu par le filtre.

La réponse Filtrat de MM-10.05 est réduite dans les cellules HEK-TLR2, mais pas dans les cellules Raw-Blue, indiquant que cet échantillon est contaminé par une association de plusieurs molécules, dont une part conséquente apporté par le PGN.

Les échantillons MM-10.04, MP-10.06 et MP-10.07 ne sont pas significativement contaminés par du PGN, puisque les réponses Totales et Filtrats sont identiques dans les cellules HEK-TLR2. Par contre, on peut noter une diminution de 50% de la réponse Filtrat pour MP-10.07 dans les cellules Raw-Blue. Le test LAL a montré que cet échantillon est chargé en LPS. Bien que la masse des endotoxines soit inférieure à 30 kDa, ces molécules sont capables de former des agrégats, ce qui peut rendre compte de la perte de réponse après filtration.

Les réponses Totales et Filtrats ont été analysées dans les types cellulaires THP-1, Raw-Blue, HEK-TLR2 et HEK-NOD2. Les résultats sont présentés dans le Tableau 4.

Pour les cellules HEK-NOD2, les réponses Totales et Filtrats sont identiques, ce qui était attendu puisque le MDP et les molécules apparentées ont une masse nettement inférieure à 30 kDa (MW∼500 Da). Seules les réponses Totales sont reprises dans le Tableau 4.

**Tableau 4 : Caractérisation des contaminants par analyse des réponses cellulaires. Les taux de contaminations sont exprimés en fonction des seuils limites de détection (SDL) et des EC50 définis dans les exemples 2 et 3 pour chaque type cellulaire (Tableaux 2 et 3), avec les courbes doses-réponses au LPS pour les cellules THP-1 sensibilisées, au PGN pour les lignées Raw-Blue et HEK-TLR2, et au MDP pour la lignée HEK-NOD2 : (-) : taux < SDL; (±) : SDL ≤ taux < 3 x SDL ; (+) : 3 x SDL ≤ taux < 0,3 x EC50 ; (++) : 0,3 x EC50 ≤ taux < 3 x EC50 ; (+++) ; taux ≥ 3 x EC50.**

| Réponse | I-10.01 | I-10.02 | I-10.03 | I-11.12 | MM-10.04 | MM-10.05 | MP-10.06 | MP-10.07 |
|---|---|---|---|---|---|---|---|---|
| THP-1 | - | - | ± | ± | + | ± | ± | + |
| < 30 kDa | - | - | ± | ± | ± | ± | ± | ± |
| Raw-Blue | - | + | ± | + | ± | + | ± | + |
| < 30 kDa | - | - | - | - | ± | ± | ± | + |
| HEK-TLR2 | - | ++ | ± | +++ | ± | ++ | ± | ± |
| < 30 kDa | - | - | ± | - | ± | ± | ± | ± |
| HEK-NOD2 | - | - | ± | - | ± | - | + | ++ |
| Effet majeur | négatif | PGN | résidus | PGN | résidus dont MDP | PGN et résidus | résidus dont MDP | LPS et résidus |

Les données permettent de caractériser les types de contaminants présents dans chaque solution de polymère de glucose et d'établir leur part dans la réponse inflammatoire :
I-10.01 : échantillon non contaminé.
I-10.02 : échantillon fortement contaminé par du PGN. L'absence de réponse des cellules THP-1, HEK-NOD2 et des Filtrats indiquent que la part des autres contaminants est négligeable.
I-10.03 : échantillon faiblement contaminé par des résidus provenant probablement de produits de dégradation de petite taille. En effet, les réponses Totales et Filtrats sont à la limite du bruit de fond dans tous les tests.
I-11.12 : échantillon fortement contaminé par du PGN. La faible réponse des cellules THP-1 est également en faveur de traces de LPS.
MM-10.04 : échantillon faiblement contaminé par du LPS et des molécules de petite taille activatrices de TLR2 (LTA, lipopeptides). La présence de MDP est également en faveur de produits de dégradation du PGN.
MM-10.05 : échantillon contaminé par du PGN et d'autres molécules de petites tailles. Les faibles réponses des autres tests suggèrent la présence de traces de LPS et d'autres ligands de TLR2.
MP-10.06 : échantillon contaminé par de nombreuses petites molécules. Par contre, les faibles réponses THP-1 et HEK-TLR2 indiquent l'absence de PGN et de LPS.
MP-10.07 : échantillon contaminé par de nombreuses petites molécules, avec une forte proportion de LPS et de MDP.

On peut noter que les résultats sont en adéquation avec les dosages SLP-HS WAKO pour les échantillons I-10.01, I-10.02, I-10.03 et I-11.12, qui sont des produits finis, et MM-10.04 et MP-10.06, qui ont été identifiés comme étant dépourvus de PGN .

Par contre, les deux échantillons MM-10.05 et MP-10.07 donnent des réponses plus faibles en PGN que celles attendues avec les données des tests SLP. Il est toutefois possible que ces échantillons aient donné des faux positifs avec les tests SLP, par réaction croisée avec des β-glucanes par exemple. Une autre possibilité est que ces échantillons contiennent de très gros PGN, dont la faible solubilité empêche le déclenchement d'une réponse dans les tests cellulaires.

### Exemple 5 : Méthode de dosage des peptidoglycanes

Le dosage est basé sur la reconnaissance spécifique des PGN par une lignée exprimant le récepteur TLR2 et sur la production d'une activité enzymatique mesurable via l'activation de la voie de signalisation associée à TLR2.

### Procédures expérimentales

Pour les expériences relatives à ce dosage, deux lignées sont utilisées :
- lignée HEK-Blue™ hTLR2 :
   - lignée HEK-Blue™ Null2:
      Les deux lignées sont présentées dans l'exemple 3

### Etablissement de la courbe doses-réponses

La courbe doses-réponses a été réalisée avec du PGN standard de *S. aureus* (Figure 21).

Les cellules HEK-Blue™ sont incubées avec des concentrations croissantes en standard, et la réponse cellulaire est mesurée par quantification de l'activité enzymatique produite.

Le résultat est une courbe classique de réponse cellulaire de type sigmoïde.
- la partie (A) correspond aux réponses obtenues avec des concentrations faibles en PGN, en-deçà de celles donnant une activation efficace de TLR2. Cette zone non linéaire correspond donc au seuil limite de détection de la méthode. De façon à inclure la variabilité de la méthode, on estime ce seuil de détection à trois fois la valeur du bruit de fond (réponse obtenue en absence de stimulus).
- la partie (B) est la plus intéressante car on observe une réponse linéaire. Cette zone de réponse efficace permet de déterminer une relation directe entre la réponse cellulaire et le taux de PGN. Il s'agit donc de la zone de dosage.
- la partie (C) correspond à une saturation de la réponse cellulaire en présence de concentrations trop fortes en PGN. Il y a en fait une saturation des récepteurs TLR2.

La courbe standard de réponse des cellules HEK-TLR2 au PGN présente une zone de linéarité pour des concentrations comprises entre 0.07 et 10 ng/mL (soit entre 2 et 267 ng/g).

Dans le cas d'échantillons susceptibles d'être fortement contaminés en PGN, il faudra réaliser plusieurs dilutions en série de façon à toujours se localiser dans la zone de linéarité. A l'inverse, des concentrations faibles en PGN nécessitent une étape de concentration de l'échantillon si l'on veut augmenter la sensibilité du dosage.

### Préparation des échantillons

Les dosages des PGN sont réalisés sur des solutions de polymères de glucose. L'échantillon nécessitant la quantification de PGN est incubé avec des cellules HEK-Blue™ hTLR2, et la réponse cellulaire est mesurée par quantification de l'activité enzymatique produite. En se reportant à la courbe doses-réponses, la quantité de PGN contenu dans l'échantillon peut être déterminée.

Les premiers tests ont été réalisés avec des échantillons contaminés provenant de lots de polymères de glucose manufacturés (Exemple 3, Figure 15).

Les cellules HEK-TLR2 permettent de détecter la présence de PGN dans la majorité des échantillons. En revanche, nous avons observé qu'il n'y a pas de corrélation entre les taux de PGN mesurés par la méthode SLP-Wako et les amplitudes des réponses cellulaires au PGN. En effet, les échantillons les plus fortement chargés en PGN ne sont pas ceux qui induisent la plus forte production de SEAP.

A titre d'illustration, l'échantillon MP-10.07, qui est le plus chargé en PGN (645 ng/mg), ne donne pas de réponse cellulaire marquée avec les cellules HEK-TLR2. A l'inverse, les échantillons I-10.02 et I-11.12 sont moins contaminés (253 et 393 ng/g, respectivement) mais sont les plus réactifs en réponse cellulaire.

Les PGN sont très hétérogènes en taille. Les formes les plus lourdes (> 10⁶ Da) sont peu solubles et donc peu réactives dans les tests cellulaires. Par contre, elles sont dosées par le test SLP-Wako. Les PGN de taille intermédiaire (∼ 100 kDa) sont très solubles et sont de puissants inducteurs de TLR2. Malgré des taux faibles, ils sont susceptibles de déclencher une forte réponse inflammatoire.. Cette dispersion de taille et donc d'activité présente un inconvénient majeur pour faire la relation entre le taux de contamination et le risque de développer une réponse inflammatoire lorsque des dosages quantitatifs classiques sont utilisés (LAL et SLP-Wako).

Les PGN présents dans les échantillons I-10.02 et I-11.12 sont solubles et donc très réactifs. A l'inverse, l'échantillon MP-10.07 contient probablement des PGN de grande taille, qui seront éliminés en cours de production du produit final.

Sur la base de ces premiers résultats, la méthode de dosage des PGN basée sur l'utilisation des cellules HEKTLR2 permettrait donc de quantifier les PGN biologiquement actifs susceptibles de provoquer des réactions inflammatoires in vivo.

Une procédure particulièrement intéressante pour doser les PGN susceptibles de déclencher une réponse inflammatoire est de réduire leur taille, et donc d'augmenter leur solubilité pour le test de réponse cellulaire in vitro.

La mutanolysine est une enzyme qui, par son activité muramidase, est capable de dépolymériser les PGN. Pour tester son activité, des solutions de PGN standard (*S. aureus*) ont été préparées en diluant la molécule dans du milieu de culture en absence ou en présence du polymère I-10.01 (polymère non contaminé) aux concentrations de 7,5 et 37,5 % (poids/volume).

Les échantillons ont été traités en présence de 2500 U/mL de mutanolysine pendant 16 h à 37°C, puis ajoutés aux cellules HEK-TLR2. La réponse cellulaire a été mesurée en suivant l'activité de la SEAP produite, selon les conditions décrites dans l'exemple 3.

En absence de polymère de glucose, le traitement à la mutanolysine pendant 16 h réduit la réponse des cellules HEK-TLR2 de plus de 50 %, indiquant que la dépolymérisation a été trop forte et a réduit la réactivité du PGN vis-à-vis des cellules. A l'inverse, la présence du polymère de glucose réduit l'activité de l'enzyme, puisque la réponse des cellules est même améliorée en présence de 7,5 % de polymère, alors qu'elle est inchangée en présence de 37,5 % de polymère.

La mutanolysine seule n'induit aucune réponse cellulaire, indiquant qu'elle n'est pas contaminée et qu'elle n'a aucun effet activateur sur les cellules.

Pour vérifier l'efficacité de ce traitement, les polymères I-10.01, I-10.02, I-10.03, I-11.12, MM-10.05 et MP-10 .07 ont été dilués à la concentration de 7,5 % puis traités pendant 16 h à 37°C en absence ou en présence de 2500 U/mL de mutanolysine.

La réponse cellulaire a été induite en ajoutant 40 µL à 160 µL de suspension cellulaire (concentration finale du polymère : 15 mg/mL).

### Résultats

Les réponses des cellules HEK-TLR2 en présence des polymères non traitées sont faibles, ce qui était attendu étant donné la plus faible concentration en polymères par rapport à l'exemple 3.

Après traitement à la mutanolysine, les réponses aux solutions de polymère de glucose sont nettement augmentées (Figure 22). En outre, on peut noter que le polymère I-10.03 donne une réponse équivalente aux polymères I-10.02 et MM-10.05, alors qu'il n'était pas réactif dans les tests précédents.

Ces résultats indiquent que la mutanolysine a partiellement dépolymérisé et donc solubilisé des PGN qui étaient en tout ou partie insolubles en raison de leur taille trop importante.

Les valeurs d'absorbance ont été reportées sur les courbes étalons établies dans les mêmes conditions avec le PGN standard (Figure 23), de façon à déterminer les concentrations en PGN présents dans les polymères de glucose.

Les résultats exprimés en ng de PGN / g de polymère de glucose sont reportés dans le tableau 5. Après traitement à la mutanolysine, les valeurs sont plus faibles que celles obtenues avec les tests SLP-Wako, mais elles reflètent la charge des polymères de glucose en PGN à activité pro-inflammatoire (Tableau 1).

Le polymère I-10.03 donne une valeur élevée, proche de I-10.02, après traitement à la mutanolysine. Cette donnée est intéressante car les deux polymères avaient porté réclamation en raison d'épisodes de péritonite aseptique. Le traitement à la mutanolysine de I-10.03 a donc permis de faire apparaître la charge en PGN actifs, probablement en permettant la solubilisation du contaminant.

Les valeurs des échantillons MM-10.05 et MP-10.07 restent plus faibles que celles obtenues avec les tests SLP. Toutefois, ces échantillons sont contaminés par d'autres molécules inflammatoires que les PGN. Ces molécules, et notamment les β-glucanes, ont probablement interféré dans les dosages SLP en augmentant la réponse des tests SLP.

**Tableau 5 : Dosage des PGN présents dans les polymères de glucose avant et après traitement à la mutanolysine. Les valeurs sont exprimés en ng équivalent PGN de S. aureus / g de polymère.**

| | **I-10.01** | **I-10.02** | **I-10.03** | **I-11.12** | **MM-10.05** | **MP-10.07** |
|---|---|---|---|---|---|---|
| **sans traitement** | **< 2** | **13,5** | **< 2** | **17,5** | **11,5** | **3,5** |
| **avec traitement** | **< 2** | **33,5** | **36,8** | **113,5** | **36** | **13,5** |

### La description concerne également les objets suivants :

1. Procédé de détection des contaminants pro-inflammatoires de polymères de glucose, lesdits contaminants étant susceptibles de déclencher, séparément ou en combinaison, une réaction inflammatoire, caractérisé en ce qu'il comprend au moins un test de réponse inflammatoire *in vitro* à l'aide d'une lignée cellulaire permettant de détecter au moins un facteur de la réponse inflammatoire, la lignée cellulaire étant soit un macrophage ou une lignée cellulaire différenciée en macrophage, soit une cellule exprimant un ou plusieurs récepteurs TLR (Toll Like Receptor) ou NOD tels que TLR2, TLR4 ou NOD2 et permettant de détecter les réponses du ou des récepteurs, soit une combinaison de celles-ci.
2. Procédé selon l'objet 1, caractérisé en ce que les polymères de glucose sont choisis dans le groupe des polymères de glucose destinés à la dialyse péritonéale, la nutrition entérale et parentérale et l'alimentation des nouveaux nés, et sont plus particulièrement les polymères de glucose destinés à la dialyse péritonéale.
3. Procédé selon l'objet 1 ou 2, caractérisé en ce que la lignée cellulaire est un macrophage, de préférence une lignée cellulaire différenciée en macrophage, de manière plus préférée la lignée THP-1 différenciée en macrophage.
4. Procédé selon l'objet 3, caractérisé en ce que la lignée cellulaire est mise en oeuvre à une densité comprise entre 0,5 à 1 x 10⁶ cellules/mL, de préférence comprise entre 0,7 à 0,8 x 10⁶ cellules/mL, et de manière encore plus préférée environ 0,75 x 10⁶ cellules/mL.
5. Procédé selon l'un quelconque des objets 3 ou 4, caractérisé en ce que le test de réponse inflammatoire consiste à mettre les cellules de la lignée cellulaire en présence d'une préparation de polymères de glucose susceptibles de contenir des contaminants pro-inflammatoires et à mesurer la production de cytokines de la phase aigue de l'inflammation, la production de ces cytokines indiquant que la préparation contient des contaminants susceptibles de déclencher une réaction inflammatoire.
6. Procédé selon l'objet 5, caractérisé en ce que les cytokines de la phase aigue de l'inflammation sont choisies dans le groupe constitué de TNF-α, IL-1β et des chimiokines telles que CCL5/RANTES et II-8/CXCL8, de préférence la chimiokine est RANTES.
7. Procédé selon l'objet 5 ou 6, caractérisé en ce que la cytokine est RANTES.
8. Procédé selon l'objet 6 ou 7, caractérisé en ce qu'on mesure la production de cytokines après 20 h de stimulation pour RANTES et/ou 8 h de stimulation pour TNF-α.
9. Procédé selon l'un quelconque des objets 5 à 8, caractérisé en ce qu'on ajoute à la préparation de polymères de glucose une molécule choisie parmi le muramyl-dipeptide (MDP) et des molécules apparentées (L18-MDP, glycolyl-MDP), des peptides microbiens formylés de type f-MLP (tripeptide formyl-Met-Leu-Phe) et des lipopolysaccharides (LPS), de préférence du MDP de *S. aureus,* du fMLP ou du LPS purifié d'Escherichia coli.
10. Procédé selon l'un quelconque des objets 5 à 9, caractérisé en ce qu'on ajoute à la préparation de polymères de glucose une molécule choisie parmi du MDP et du LPS.
11. Procédé selon l'objet 10, caractérisé en ce qu'on ajoute à la préparation de polymères de glucose du MDP à une concentration comprise entre 1 et 100 µg/mL, de préférence d'environ 10 µg/mL.
12. Procédé selon l'un quelconque des objets 3 à 11, caractérisé en ce que le procédé présente une ou plusieurs des caractéristiques suivantes, de préférence toutes les caractéristiques, :
   - la lignée cellulaire est mise en oeuvre à une densité comprise entre 0,5 à 1 x 10⁶ cellules/mL, de préférence comprise entre 0,7 à 0,8 x 10⁶ cellules/mL, et de manière encore plus préférée environ 0,75 x 10⁶ cellules/mL; et/ou
   - la concentration de polymère de glucose est inférieure à 50 mg/ml, de préférence environ 25 mg/ml ; et/ou
   - on mesure la production de cytokines après 20 h de stimulation pour RANTES et/ou 8 h de stimulation pour TNF-α; et/ou
   - on ajoute à la préparation de polymères de glucose du MDP à une concentration comprise entre 1 et 100 µg/mL, de préférence d'environ 10 µg/mL.
13. Procédé selon l'un quelconque des objets 3 à 12, caractérisé en ce qu'il permet de détecter la contamination du polymère de glucose par des peptidoglycanes (PGN) et/ou LPS, de préférence par des PGN de taille moyenne d'environ 120 kDa et/ou LPS, plus particulièrement encore par des LPS.
14. Procédé selon l'objet 1 ou 2, caractérisée en ce que la lignée cellulaire permet de détecter l'activité d'un ou plusieurs récepteurs TLR (Toll Like Receptor) ou NOD tels que TLR2, TLR4 ou NOD2 et en ce que, dans la lignée cellulaire, un gène rapporteur est sous la dépendance directe de la voie de signalisation associée au(x) récepteur(s) TLR ou NOD, de préférence un gène rapporteur codant pour une protéine colorée ou fluorescente ou pour une protéine dont l'activité peut être mesurée avec ou sans substrat, de manière encore plus préférée pour une phosphatase alcaline sécrétée.
15. Procédé selon l'objet 14, caractérisée en ce que la lignée cellulaire permet de détecter l'activité d'un récepteur TLR2, notamment la lignée HEK-Blue™ hTLR2.
16. Procédé selon l'objet 14, caractérisée en ce que la lignée cellulaire permet de détecter l'activité d'un récepteur NOD2, notamment la lignée HEK-Blue™ hNOD2.
17. Procédé selon l'objet 14, caractérisée en ce que la lignée cellulaire permet de détecter l'activité d'un récepteur TLR4, notamment la lignée HEK-Blue™ hTLR4.
18. Procédé selon l'objet 14, caractérisée en ce que la lignée cellulaire permet de détecter l'activité des récepteurs TLR2, TLR4 et NOD2, notamment la lignée Raw-Blue™.
19. Procédé selon l'un quelconque des objets 14 à 18, caractérisé en ce que le test de réponse inflammatoire consiste à mettre les cellules de la lignée cellulaire en présence d'une préparation de polymères de glucose susceptibles de contenir des contaminants pro-inflammatoires et à mesurer l'activité du récepteur ou le signal du gène rapporteur, la détection de cette activité ou de ce signal indiquant que la préparation contient des contaminants susceptibles d'activer un ou des récepteurs de l'immunité innée et de déclencher une réaction inflammatoire.
20. Procédé selon l'objet 16, caractérisé en ce que le précédé permet de détecter la contamination du polymère de glucose par des MDP.
21. Procédé selon l'objet 15 ou 18, caractérisé en ce que le précédé permet de détecter la contamination du polymère de glucose par des PGN.
22. Procédé selon l'un quelconque des objets 1 à 21, caractérisé en ce que la préparation de polymères de glucose testée présente une concentration de polymères de 5 à 50 mg/mL.
23. Procédé selon l'un quelconque des objets 1 à 22, caractérisé en ce qu'il comprend une étape de traitement de la préparation de polymères de glucose par une mutanolysine préalable à l'incubation de ladite préparation avec les cellules.
24. Procédé selon l'un quelconque des objets 1 à 22, caractérisé en ce qu'il comprend en outre une étape de filtration de la préparation de polymères de glucose avec un seuil de coupure compris entre 30 kD et 150 kD, de préférence entre 30 et 100 kD ou entre 30 et 50 kD, et notamment d'environ 30 kD, et en ce que le filtrat est incubé avec les cellules, et facultativement en ce que les résultats sur le filtrat sont comparés aux résultats sur la préparation de polymères de glucose.
25. Procédé selon l'un quelconque des objets 1 à 24, caractérisé en ce que le procédé selon l'invention comprend les étapes consistant à
   (a) réaliser au moins un test de réponse inflammatoire *in vitro* consistant à mettre des macrophages en présence d'une préparation de polymères de glucose susceptibles de contenir des contaminants pro-inflammatoires et à mesurer la production de cytokines de la phase aigue de l'inflammation, notamment TNF-α ou RANTES, de préférence RANTES, la production de ces cytokines indiquant que la préparation contient des contaminants susceptibles de déclencher une réaction inflammatoire, et
   (b) mettre en présence une lignée cellulaire permettant de détecter l'activité d'un récepteur ou plusieurs récepteurs de l'immunité innée telle que TLR2, TLR4 ou NOD2, avec la préparation et détecter l'activité du récepteur ou le signal du gène rapporteur associé à ce récepteur, la détection de cette activité ou de ce signal indiquant la présence dans la préparation d'un contaminant qui est un agoniste du récepteur.
26. Procédé selon l'objet 25, caractérisé en ce qu'il comprend la réalisation d'un test avec des macrophages (a) et d'un test (b) avec des cellules permettant de détecter l'activité du récepteur TLR2, et facultativement d'un test (b) avec des cellules permettant de détecter l'activité du récepteur NOD2 et/ou d'un test (b) avec des cellules permettant de détecter l'activité des récepteurs TLR2, TLR4 et NOD2 ; de préférence il comprend la réalisation d'un test avec des macrophages (a) et d'un test (b) avec des cellules permettant de détecter l'activité du récepteur TLR2 telles que HEK-Blue™ hTLR2, des cellules permettant de détecter l'activité du récepteur NOD2 telles que HEK-Blue™ hNOD2 et, des cellules permettant de détecter l'activité des récepteurs TLR2, TLR4 et NOD2 telles que Raw-Blue™.
27. Procédé selon l'un quelconque des objets 1 à 26, caractérisé en ce qu'il comprend en outre une étape consistant à identifier ou à quantifier le(s) contaminant(s) susceptible(s) de déclencher une réaction inflammatoire.
28. Procédé selon l'objet 27, caractérisé en ce que le(s) contaminant(s) susceptible(s) de déclencher une réaction inflammatoire sont identifiés ou quantifiés en mettant la préparation de polymères de glucose en présence des cellules d'une lignée cellulaire telle que définie dans l'une quelconque des revendications 14 à 18 et en mesurant l'activité du récepteur ou le signal du gène rapporteur, la détection de cette activité ou de ce signal indiquant la présence dans la préparation d'un contaminant qui est un agoniste du récepteur.
29. Procédé selon l'objet 28, caractérisé en ce que le procédé permet la quantification de peptidoglycanes (PGN) dans lequel la lignée cellulaire mise en oeuvre permet de détecter l'activité d'un récepteur TLR2, notamment via un gène rapporteur sous la dépendance directe de la voie de signalisation associée au récepteur de l'immunité TLR2.
30. Procédé selon l'objet 29, caractérisé en ce que le procédé comprend une étape de traitement de la préparation de polymères de glucose par une mutanolysine, l'incubation des préparations de polymères de glucose traitée par une mutanolysine et/ou non traitée et la quantification des PGN.

## Revendications

1. Procédé de détection des contaminants pro-inflammatoires de polymères de glucose, les dits contaminants étant susceptibles de déclencher, séparément ou en combinaison, une réaction inflammatoire **caractérisé en ce que** le procédé selon l'invention comprend les étapes consistant à
a) réaliser au moins un test de réponse inflammatoire *in vitro* consistant à mettre un macrophage ou une lignée cellulaire différenciée en macrophage en présence d'une préparation de polymères de glucose susceptibles de contenir des contaminants pro-inflammatoires et à mesurer la production de cytokines de la phase aigue de l'inflammation, notamment TNF-α ou RANTES, de préférence RANTES, la production de ces cytokines indiquant que la préparation contient des contaminants susceptibles de déclencher une réaction inflammatoire, et
b) mettre en présence une lignée cellulaire permettant de détecter l'activité d'un récepteur ou plusieurs récepteurs de l'immunité innée telle que TLR2, TLR4 ou NOD2, avec la préparation et détecter l'activité du récepteur ou le signal du gène rapporteur associé à ce récepteur, la détection de cette activité ou de ce signal indiquant la présence dans la préparation d'un contaminant qui est un agoniste du récepteur.

2. Procédé selon la revendication 1, **caractérisé en ce qu'**il comprend la réalisation d'un test avec des macrophages (a) et d'un test (b) avec des cellules permettant de détecter l'activité du récepteur TLR2, et facultativement d'un test (b) avec des cellules permettant de détecter l'activité du récepteur NOD2 et/ou d'un test (b) avec des cellules permettant de détecter l'activité des récepteurs TLR2, TLR4 et NOD2 ; de préférence il comprend la réalisation d'un test avec des macrophages (a) et d'un test (b) avec des cellules permettant de détecter l'activité du récepteur TLR2 telles que HEK-Blue™ hTLR2, des cellules permettant de détecter l'activité du récepteur NOD2 telles que HEK-Blue™ hNOD2 et, des cellules permettant de détecter l'activité des récepteurs TLR2, TLR4 et NOD2 telles que Raw-Blue™.

3. Procédé selon l'une quelconque des revendications 1 à 2, **caractérisé en ce que** la lignée cellulaire différenciée en macrophage est la lignée THP-1 différenciée en macrophage.

4. Procédé selon l'une quelconque des revendications 1 à 3, **caractérisé en ce que** les polymères de glucose sont choisis dans le groupe des polymères de glucose destinés à la dialyse péritonéale, la nutrition entérale et parentérale et l'alimentation des nouveaux nés, et sont plus particulièrement les polymères de glucose destinés à la dialyse péritonéale.

5. Procédé selon l'une quelconque des revendications 1 à 4, **caractérisé en ce qu'**on ajoute à la préparation de polymères de glucose une molécule choisie parmi le muramyl-dipeptide (MDP) et des molécules apparentées (L18-MDP, glycolyl-MDP), des peptides microbiens formylés de type f-MLP (tripeptide formyl-Met-Leu-Phe) et des lipopolysaccharides (LPS), de préférence du MDP de S. aureus, du fMLP ou du LPS purifié d'Escherichia coli, de manière encore plus préférée une molécule choisie parmi du MDP et du LPS.

6. Procédé selon l'une quelconque des revendications 1 à 5, **caractérisé en ce que** le procédé présente une ou plusieurs des caractéristiques suivantes, de préférence toutes les caractéristiques :
- la lignée cellulaire est mise en oeuvre à une densité comprise entre 0,5 à 1 x 10⁶ cellules/mL, de préférence comprise entre 0,7 à 0,8 x 10⁶ cellules/mL, et de manière encore plus préférée environ 0,75 x 10⁶ cellules/mL; et/ou
- la concentration de polymère de glucose est inférieure à 50 mg/ml, de préférence environ 25 mg/ml ; et/ou
- on mesure la production de cytokines après 20 h de stimulation pour RANTES et/ou 8 h de stimulation pour TNF-α ; et/ou
- on ajoute à la préparation de polymères de glucose du MDP à une concentration comprise entre 1 et 100 µg/mL, de préférence d'environ 10 µg/mL.

7. Procédé selon l'une quelconque des revendications 1 à 6, **caractérisé en ce qu'**il permet de détecter la contamination du polymère de glucose par des peptidoglycanes (PGN) et/ou LPS, de préférence par des PGN de taille moyenne d'environ 120 kDa et/ou LPS, plus particulièrement encore par des LPS.

8. Procédé selon l'une quelconque des revendications 1 à 6, **caractérisé en ce que** le précédé permet de détecter la contamination du polymère de glucose par des MDP.

9. Procédé selon l'une quelconque des revendications 1 à 8, **caractérisée en ce que** la lignée cellulaire permet de détecter l'activité d'un ou plusieurs récepteurs TLR (Toll Like Receptor) ou NOD tels que TLR2, TLR4 ou NOD2 et **en ce que**, dans la lignée cellulaire, un gène rapporteur est sous la dépendance directe de la voie de signalisation associée au(x) récepteur(s) TLR ou NOD, de préférence un gène rapporteur codant pour une protéine colorée ou fluorescente ou pour une protéine dont l'activité peut être mesurée avec ou sans substrat, de manière encore plus préférée pour une phosphatase alcaline sécrétée.

10. Procédé selon l'une quelconque des revendications 1 à 9, **caractérisée en ce que** la lignée cellulaire permet de détecter :
- l'activité d'un récepteur TLR2, notamment la lignée HEK-BlueTM hTLR2 ou
- l'activité d'un récepteur NOD2, notamment la lignée HEK-BlueTM hNOD2 ou
- l'activité d'un récepteur TLR4, notamment la lignée HEK-BlueTM hTLR4 ou
- l'activité des récepteurs TLR2, TLR4 et NOD2, notamment la lignée Raw-BlueTM.

11. Procédé selon l'une quelconque des revendications 1 à 10, **caractérisé en ce qu'**il comprend en outre une étape de filtration de la préparation de polymères de glucose avec un seuil de coupure compris entre 30 kD et 150 kD, de préférence entre 30 et 100 kD ou entre 30 et 50 kD, et notamment d'environ 30 kD, et **en ce que** le filtrat est incubé avec les cellules, et facultativement **en ce que** les résultats sur le filtrat sont comparés aux résultats sur la préparation de polymères de glucose.

12. Procédé selon l'une quelconque des revendications 1 à 11, **caractérisé en ce qu'**il comprend en outre une étape consistant à identifier ou à quantifier le(s) contaminant(s) susceptible(s) de déclencher une réaction inflammatoire.

13. Procédé selon la revendication 12, **caractérisé en ce que** le(s) contaminant(s) susceptible(s) de déclencher une réaction inflammatoire sont identifiés ou quantifiés en mettant la préparation de polymères de glucose en présence des cellules d'une lignée cellulaire telle que définie dans l'une quelconque des revendications 9 à 10 et en mesurant l'activité du récepteur ou le signal du gène rapporteur, la détection de cette activité ou de ce signal indiquant la présence dans la préparation d'un contaminant qui est un agoniste du récepteur.

14. Procédé selon la revendication 13, **caractérisé en ce que** le procédé permet la quantification de peptidoglycanes (PGN) dans lequel la lignée cellulaire mise en oeuvre permet de détecter l'activité d'un récepteur TLR2, notamment via un gène rapporteur sous la dépendance directe de la voie de signalisation associée au récepteur de l'immunité TLR2.

15. Procédé selon la revendication 14, **caractérisé en ce que** le procédé comprend une étape de traitement de la préparation de polymères de glucose par une mutanolysine, l'incubation des préparations de polymères de glucose traitée par une mutanolysine et/ou non traitée et la quantification des PGN.
